# EUROPEAN PATENT APPLICATION

(11) **EP 4 095 151 A2**
(43) Date of publication of application: **30.11.2022**
(21) Application number: 22166183.8
(22) Date of filing: 31.03.2022
(51) Int. Cl.: C07K 14/245, C07K 14/395, C12P 7/46, C12R 1/865

(54) **YEAST CELL EXPRESSING A PLASMA MEMBRANE ORGANIC ACID TRANSPORTER, METHODS AND USES THEREOF**

(30) Priority: 26.05.2021 PT 2021117253
(71) Applicant: Universidade do Minho, 4704-553 Braga (PT)
(72) Inventor: PEDRA AMORIM CASAL, MARGARIDA PAULA, 4715-123 BRAGA (PT); SOARES DA SILVA, ISABEL JOÃO, 4715-027 BRAGA (PT); RENDULIC, TONI, 4710-407 BRAGA (PT)
(74) Representative: Patentree

(57) **Abstract**

The present disclosure relates to a yeast cell comprising an expression cassette for expressing a functional SatP transmembrane transporter of organic acids, and also to a yeast cell comprising an expression cassette for expressing a functional modified SatP and/or Ato1 transmembrane transporter of succinic acid. The use of a yeast cell comprising a SatP or Ato1 transmembrane transporter as a succinic acid transporter, and the use of a yeast cell comprising a SatP or Ato1 transmembrane transporter as an improver of succinic acid production or consumption are also disclosed.

## Description

### TECHNICAL FIELD

The present disclosure relates to a yeast cell, expressing a plasma membrane organic acid transporter, in particular a plasma membrane transporter for succinic acid.

### BACKGROUND

*Saccharomyces cerevisiae* is a common cell factory for organic acid production due to its tolerance to acidic conditions and extensive knowledge of its molecular genetics, physiology and genomics, making it a very attractive platform for metabolic engineering. One of the major bottlenecks for the efficient and cost-effective bioproduction is the export of organic acids through the microbial plasma membrane (1). So far four native organic acid plasma membrane transporters were functionally characterized in *S*. *cerevisiae* (2): the lactate transporter Jen1, TC #2.A.1.12.2, the acetate transporter Ato1 (previously known as Ady2), TC #2.A.96.1.4, the acetic acid channel Fps1, TC #1.A.8.5.1 and the sorbate/benzoate pump Pdr12, TC #3.A.1.205.3.

The scientific community and the biotech industry are currently focused on the development of robust microbial cell factories for efficient organic acid production from cheap industrial biowastes. Since the recognition that organic acid export over the plasma membrane represents one of the key steps in microbial production of these compounds, organic acid transporters started receiving greater attention in metabolic engineering strategies (1). Efforts aimed at the identification of biotechnologically relevant membrane transporters and their further improvement by the means of transporter engineering will lead towards more efficient biotechnological processes, however, more structure-function relationship models are needed to boost the emerging field of transporter engineering (1). Many efforts have been made to engineer efficient bio-production of succinic acid (1) and efficient succinic acid exporters are crucial to achieve high titres of this acid by yeast cell factories. Currently, industrial strains were engineered for the expression of transporters SpMae1 and AnDCT-02, both described as anion channels (1) that promote succinate export, allowing the production of high succinic acid titres at low pH (1,3).

Ato1 and its homologues Ato2 and Ato3 belong to the Acetate Uptake Transporter (AceTr) family whose members are distinguishable by the NPAPLGL(M/S) signature motif and are present in a wide range of organisms, namely bacteria, archaea, and fungi (4). Ato1 is a plasma membrane transporter from the model organism *S*. *cerevisiae,* and a member of the Acetate Uptake Transporter (AceTr) family, TC #2.A.96. Members of the AceTr family are greatly conserved in fungi as they were detected in 97% of available fungal genomes and presumably play an important role in spore germination within this eukaryotic kingdom (2,4). Although first identified as the main transporter responsible for the uptake of acetic acid into the cytosol (2), subsequent studies demonstrated that Ato1 could transport organic acids in both directions, as it was involved in the export of lactic acid from *S*. *cerevisiae* cells engineered for lactic acid production (1). Its export capabilities make Ato1 an interesting engineering target for organic acid production in yeast cell factories.

Acetate is the main substrate of functionally characterized transporters from the AceTr family (4), however, members of this family can also transport other organic acids. Ato1 shows transport activity for other monocarboxylates along acetate, such as formate, propionate, lactate, and pyruvate (2) while the bacterial homologue SatP, besides acetate and lactate, can also transport dicarboxylates such as succinate (5). The *ATO1*^{C655G} and *ATO1*^{C755G} alleles with higher lactate transport efficiency were obtained in a laboratory evolution experiment, causing L219V and A252G amino acid substitutions, respectively, and equivalent amino acid substitutions L131V and A164G increased the lactate transport activity of SatP in *E. coli* (5). The crystal structures of bacterial homologues SatP_Ec and SatP_Ck depict hexameric anion channels in their closed state (6). Each monomer features 6 transmembrane helices and four acetate binding sites lined up along the pore: S1 at the cytoplasmic vestibule, S2 and S3 within the main pore and S4 at the periplasmic vestibule. The structure of the SatP_Ec monomer resembles an hourglass-like shape, with a constrictive site in the pore centre defined by three conserved hydrophobic residues F17, Y72 and L131, thus termed FLY (6). These studies provided vital information regarding the physiological function, phylogeny, and structure of transporters from the AceTr family. However, more investigation is required to unveil the details of substrate translocation (6) including the ability of Ato1 (L219V & A252G) and SatP (L131V & A164G) mutants to transport lactic acid (5). Severe acetic acid sensitivity was observed in *Yarrowia lypolytica* associated with mutations in *GPR1,* a member of the AceTr family. The effect of *GPR1^{d}* alleles was dominant negative since the co-presence of wild-type *GPR1* could not alleviate sensitive phenotypes (7). The sequenced *GPR1^{d}* alleles contained mutations that led to single amino acid substitutions G62S, G63D, L65Q and G248D and were able to trans-dominantly induce acetic acid sensitivity in *S*. *cerevisiae.* Furthermore, single amino acid substitutions A70V, F71V, T74I, A88V, E144G, N145D, M211K, and F212S within Ato1 and N75D, G147D, and S265L within Ato2 were discovered to produce the same hypersensitive effect in *S. cerevisiae,* showing that induction of acetic acid sensitivity is not exclusive for *GPR1* mutants. While specific single amino acid substitutions in Gpr1, Ato1 and Ato2 were able to cause such drastic inhibitory effects, surprisingly, knockout of *GPR1* in *Y. lipolytica* or triple knockout of *ATO1, ATO2* and *ATO3* in *S*. *cerevisiae* did not cause any phenotypic changes (7).

The toxic effects of organic acids on microbial cell growth are a well-known phenomenon typically caused by the passive diffusion of undissociated form of the acids into the cytosol. Inside the cell, such organic acids predominantly dissociate into protons and anions because their p*K_{α}* values are lower than the near-neutral cytosolic pH value. The released protons acidify the cytosol and disturb cell homeostasis, while anions may produce specific toxic effects on the metabolism depending on the organic acid in play (2).

These facts are disclosed in order to illustrate the technical problem addressed by the present disclosure.

### GENERAL DESCRIPTION

The present disclosure relates to a yeast cell transformed for the expression of the Ato1 mutant proteins, able to transport an organic acid across the plasma membrane, the sequence of amino acids of the mutant proteins, the method of improving the transport across the plasma membrane of an organic acid using a yeast cell. Briefly, an organic acid is an organic compound with acidic properties. The most common organic acids are the carboxylic acids, such as acetic acid, lactic acid, pyruvic acid, malic acid, succinic acid.

The present disclosure also relates to a yeast cell transformed for the expression of the bacterial transporter SatP protein, the sequence of amino acids of the protein, the method of expressing a bacterial plasma membrane transporter protein in a yeast cell.

An aspect of the present disclosure relates to a yeast cell transformed for the expression of the bacterial SatP mutants, able to transport an organic acid across the plasma membrane, the sequence of amino acids of the mutant proteins, the method of improving the transport across the plasma membrane of an organic acid using a yeast cell.

In an embodiment, the rational design of mutations in the FLY constrictive site, altering the diameter of the pore, had an impact on transporter specificity and activity. In an embodiment, the cells' hypersensitivity to organic acids is a phenotype associated with mutants which possess increased transport activity. In an embodiment, the codon optimized *Escherichia coli* SatP protein was properly expressed in *S. cerevisiae* cells and was able to mediate the uptake of organic acids.

The present disclosure relates to a yeast cell comprising an expression cassette for expressing a functional SatP transmembrane transporter of organic acids.

In an embodiment, the expression cassette comprises at least a sequence 90% identical to a sequence of the following list: SEQ ID No 94, SEQ ID No 95, or mixtures thereof. Preferably, the expression cassette comprises at least a DNA sequence 91%, 92%, 93%, 94%, 95%, 96%, 97%, 98%, 99% or identical to a sequence of the following list: SEQ ID No 94, SEQ ID No 95, or mixtures thereof.

In an embodiment, the yeast cell comprises a functional transmembrane transporter protein wherein the transmembrane transporter protein comprises at least a sequence 90% identical to a sequence of the following list: SEQ ID No 103, SEQ ID No 104, or mixtures thereof. Preferably, the transmembrane transporter protein comprises at least a sequence 91%, 92%, 93%, 94%, 95%, 96%, 97%, 98%, 99% or identical to a sequence of the following list: SEQ ID No 103, SEQ ID No 104, or mixtures thereof.

In an embodiment, the transmembrane transporter is arranged to transport organic acids across the yeast cell membrane, wherein the organic acid is selected from a list comprising: acetic acid, lactic acid, pyruvic acid, malic acid, fumaric acid, succinic acid, or mixtures thereof. Preferably, the organic acid is selected from a list comprising: acetic acid, lactic acid, pyruvic acid, malic acid, succinic acid, or mixtures thereof.

The present disclosure also relates to a yeast cell comprising an expression cassette for expressing a functional SatP and/or Ato1 transmembrane transporter of succinic acid.

In an embodiment, the expression cassette comprises at least a sequence 90% identical to the DNA sequences of the following list: SEQ ID No 88, SEQ ID No 89, SEQ ID No 90, SEQ ID No 91, SEQ ID No 92, SEQ ID No 93, SEQ ID No 94, SEQ ID No 95, or mixtures thereof. Preferably, the expression cassette comprises at least a sequence 91%, 92%, 93%, 94%, 95%, 96%, 97%, 98%, 99% or identical to SEQ ID No 88, SEQ ID No 89, SEQ ID No 90, SEQ ID No 91, SEQ ID No 92, SEQ ID No 93, SEQ ID No 94, SEQ ID No 95, or mixtures thereof.

In an embodiment, the transmembrane transporter protein comprises at least a sequence 90% identical to the sequences of the following list: SEQ ID No 97, SEQ ID No 98, SEQ ID No 99, SEQ ID No 100, SEQ ID No 101, SEQ ID No 102, SEQ ID No 103, SEQ ID No 104, or mixtures thereof. Preferably, the transmembrane transporter protein comprises at least a sequence 91%, 92%, 93%, 94%, 95%, 96%, 97%, 98%, 99% or identical to SEQ ID No 97, SEQ ID No 98, SEQ ID No 99, SEQ ID No 100, SEQ ID No 101, SEQ ID No 102, SEQ ID No 103, SEQ ID No 104, or mixtures thereof.

For the scope and interpretation of the present disclosure, an expression cassette is a distinct component of a vector DNA consisting of a gene and a regulatory sequence to be expressed by a transfected cell. The expression cassette directs the cell's machinery to produce RNA and protein(s). Briefly, an expression cassette comprises a promoter sequence, an open reading frame, and a terminator.

In an embodiment, the expression cassette is a yeast promotor, an open reading frame and a yeast terminator.

In an embodiment, the yeast cell according to any of the previous claims wherein the expression cassette is comprised in a plasmid p416GPD (SEQ ID No 105).

In an embodiment, the transmembrane transporter is arranged to transport the organic acids across the yeast cell plasma membrane. In a further embodiment, the transmembrane transporter is arranged to transport the organic acids from an exterior of the transformed yeast cell to an interior of the said yeast cell. In a further embodiment, the transmembrane transporter is arranged to transport organic acids from an interior of the transformed yeast cell to an exterior of the said yeast cell.

In an embodiment, the transmembrane transporter is arranged to transport a second organic acid across the yeast cell membrane, wherein the second organic acid is selected from a list comprising: acetic acid, lactic acid, pyruvic acid, malic acid, fumaric acid, succinic acid, or mixtures thereof. Preferably, the second organic acid is selected from a list comprising: acetic acid, lactic acid, pyruvic acid, malic acid, succinic acid, or mixtures thereof.

In an embodiment the yeast belongs to the genus *Saccharomyces,* preferably *Saccharomyces cerevisiae.*

In an embodiment, the disclosed yeast cell is for succinic acid production and/or consumption.

An aspect of the present disclosure relates to a use of a yeast cell comprising a SatP transmembrane transporter as a succinic acid transporter, preferably a SatP transmembrane transporter protein comprising at least a sequence 90% identical to SEQ ID No 104; more preferably at least a sequence 91%, 92%, 93%, 94%, 95%, 96%, 97%, 98%, 99% or identical to SEQ ID No 104.

Another aspect of the present disclosure relates to a use of a yeast cell comprising an Ato1 transmembrane transporter as a succinic acid transporter, preferably a Ato1 transmembrane transporter protein comprising at least a sequence 90% identical to a sequence of the following list: SEQ ID No 97, SEQ ID No 98, SEQ ID No 99, SEQ ID No 100, SEQ ID No 101, SEQ ID No 102, or mixtures thereof; more preferably at least a sequence 91%, 92%, 93%, 94%, 95%, 96%, 97%, 98%, 99% or identical to a sequence of the following list: SEQ ID No 97, SEQ ID No 98, SEQ ID No 99, SEQ ID No 100, SEQ ID No 101, SEQ ID No 102, or mixtures thereof.

Another aspect of the present disclosure relates to a use of a yeast cell comprising a SatP transporter as an improver of succinic acid production and/or consumption, preferably a SatP transmembrane transporter protein comprising at least a sequence 90% identical to SEQ ID No 104; more preferably at least a sequence 91%, 92%, 93%, 94%, 95%, 96%, 97%, 98%, 99% or identical to SEQ ID No 104.

Another aspect of the present disclosure relates to a use of a yeast cell comprising an Ato1 transporter as an improver of succinic acid production and/or consumption, preferably a Ato1 transmembrane transporter protein comprising at least a sequence 90% identical to a sequence of the following list: SEQ ID No 97, SEQ ID No 98, SEQ ID No 99, SEQ ID No 100, SEQ ID No 101, SEQ ID No 102, or mixtures thereof; more preferably at least a sequence 91%, 92%, 93%, 94%, 95%, 96%, 97%, 98%, 99% or identical to a sequence of the following list: SEQ ID No 97, SEQ ID No 98, SEQ ID No 99, SEQ ID No 100, SEQ ID No 101, SEQ ID No 102, or mixtures thereof.

### BRIEF DESCRIPTION OF THE DRAWINGS

The following figures provide preferred embodiments for illustrating the disclosure and should not be seen as limiting the scope of invention.

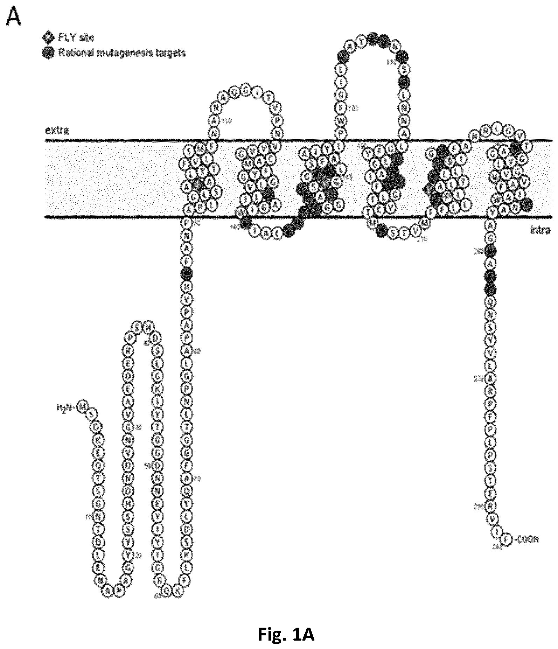

Figure 1 A-C: Screening of S. cerevisiae Ato1 mutants obtained by site-directed mutagenesis.
   A - The predicted Ato1 topology showcasing the locations of 34 amino acid residues (highlighted in red) which were substituted by alanine. The residues which form the central constrictive site (FLY) are diamond-shaped and represented by white characters.
   B - Heatmap of the 34 Ato1 mutants expressed in S. cerevisiae IMX1000 based on their effect on growth on acetic, lactic, and succinic acids as sole carbon sources. The mutant phenotypes could be grouped into 5 categories such as: reduced growth on acetic acid (Q133A, E140A, F147A, T150A, L152A, C153A, Y155A, F158A, W159A, L191A, W194A, F197A, T198A, K207A, F216A, F223A, R239A); increased ability to grow on lactic acid (N145A); gained the ability to grow both on lactic and on succinic acids (F98A, L219A); inability to grow on acetic acid (E144A); no phenotypic changes compared to wild-type Ato1-GFP (K86A, T146A, L226A, H230A, Y254A, V260A, T262A, K263A).
   C - Localization of GFP-tagged wild-type Ato1 and its mutants. Cells were collected at mid-exponential growth phase from YNB glucose 2% (w/v), washed and derepressed for 4 h in YNB lactic acid (0.5%), and observed by epifluorescence microscopy.
**Figure 2****:** Multiple sequence alignment of AceTR homologues. Residues targeted for mutation are highlighted in red. Residues corresponding to the narrowest constriction site (FLY) are highlighted in blue boxes.
**Figure 3****:** Functional analysis of the Ato1 L219A, L219G, L219V, A252G, and L219A/A252G mutants in *S. cerevisiae* IMX 1000 cells. Cells were grown on YNB media containing acetic acid (0.5%, pH 6.0) (AA), lactic acid (0.5%, pH 5.0) (LA), or succinic acid (0.5%, pH 5.0) (SA) at 18 °C for 10 days.
**Figure 4****:** Functional analysis of the Ato1 residues F98-Y155-L219.
   A - Spot assay of S. cerevisiae IMX1000 cells expressing Ato1 mutants in the residues F98 (yellow), Y155 (red), and L219 (green) grown on YNB media containing acetic acid (0.5%, pH 6.0) (AA), lactic acid (0.5%, pH 5.0) (LA), or succinic acid (0.5%, pH 5.0) at 18 °C for 10 days.
   B - Uptake of acetic acid (1.0 mM, pH 6.0), lactic acid (3.0 mM, pH 6.0) and succinic acid (2.0 mM, pH 6.0) in S. cerevisiae IMX1000 cells transformed with the plasmids p416GPD, pAdy2, and pL219A. Cells were collected at mid-exponential growth phase from YNB-glucose 2% (w/v), washed and derepressed for 4 h in YNB glycerol (6%).

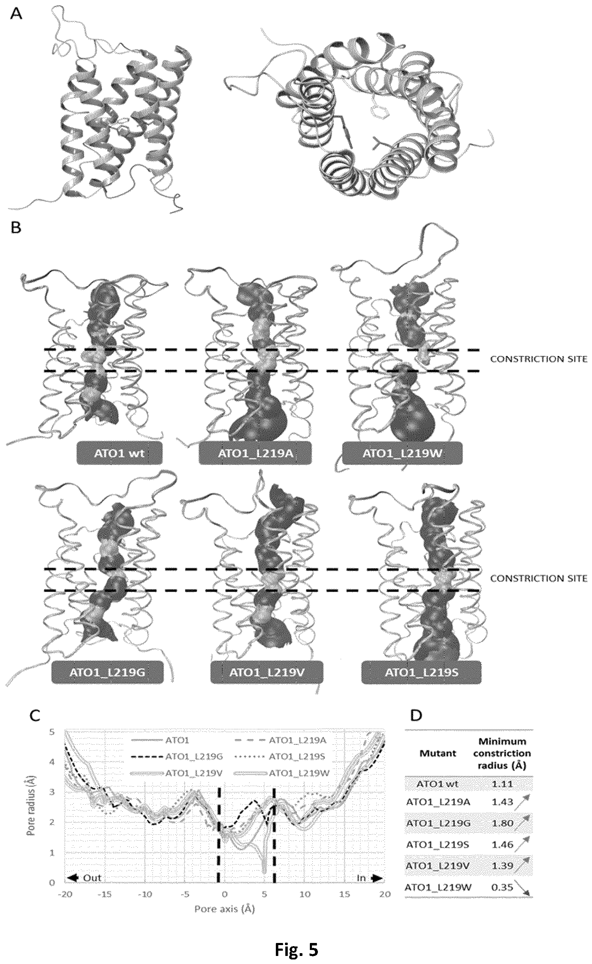

**Figure 5****:** Predicted 3D structure model of Ato1 obtained by homology threading using SatP_Ck crystal structure (PDB Entry: 5YS3) as template.
   A - Ato1 consists of six transmembrane α-helices with both termini at the intracellular side. The residues F98 (yellow), Y155 (red), and L219 (green) in the 3D structure of the Ato1 (transversal and extracellular view) form the central and narrowest hydrophobic constriction of the anion pathway. In-intracellular; Ext-extracellular.
   B - Analysis of the effect of the mutations L219A, L219G, L219S, L219V and L219W on the protein 3D structure using HOLE software (http://www.holeprogram.org).
   C - Prediction of the pore constriction along the axis of the native Ato1 and of mutations L219A, L219G, L219S, L219V and L219W using HOLE software (http://www.holeprogram.org).

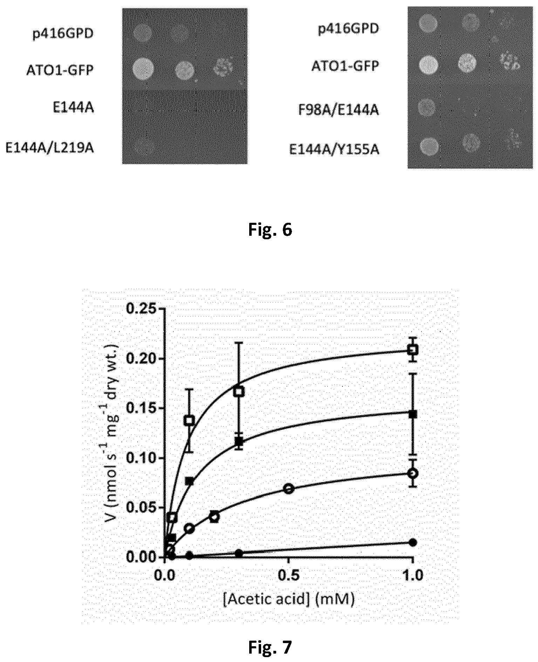

**Figure 6****:** Functional analysis of the Ato1 E144A, E144A/L219A, F98A/E144A and E144A/Y155A mutants in S. cerevisiae IMX 1000 cells. Cells were spotted on YNB media containing acetic acid (0.5%, pH 6.0) (AA) at 18°C for 10 days.
**Figure 7****:** Initial uptake rates of radiolabelled 14C-acetic acid as a function of the acid concentration in S. cerevisiae IMX1000 cells transformed with the plasmids p416GPD (full circles), pAdy2:GFP (open circles), pE144A::GFP (full squares), pSatP (empty squares). Cells were collected at mid-exponential growth phase from YNB-glucose 2% (w/v), washed and derepressed for 4 h in YNB lactic acid (0.5%, pH 5.0).

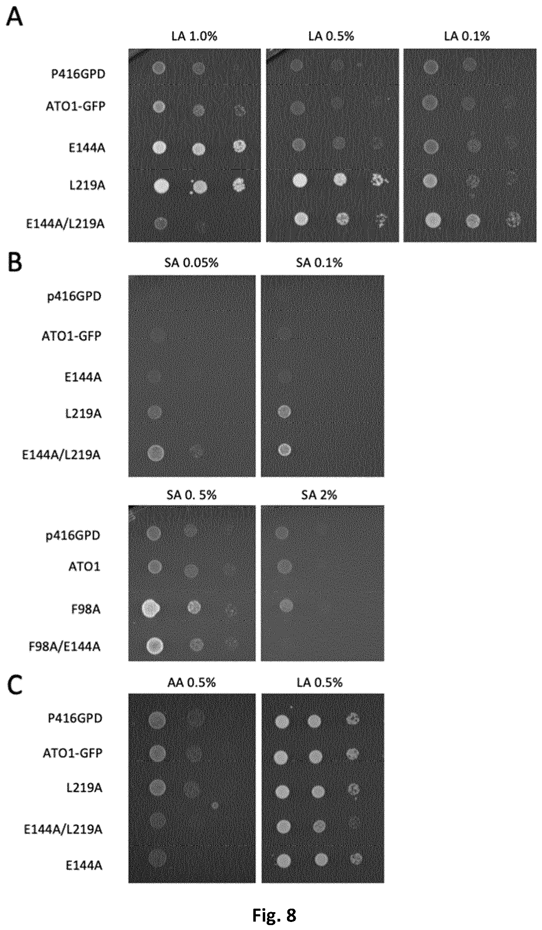

**Figure 8****:** Analysis of the hypersensitivity phenotype of Ato1 E144A allele.
   A - Spot assay of S. cerevisiae IMX1000 transformed with Ato1 mutant alleles in the residues E144, L219 and E144/L219, grown on YNB media containing 0.1%, 0.5% and 0.1% of lactic acid, pH 5.0 (LA) at 18°C for 10 days.
   B - Spot assay of S. cerevisiae IMX1000 transformed with Ato1 mutant alleles in the residues E144A, L219A, E144/L219A and F98A/E144A, grown on YNB containing 0.05%, 0.1%, 1.0% and 2.0% succinic acid, pH 5.0 (SA) at 18°C for 10 days.
   C - Spot assay of S. cerevisiae CEN.PK 113-5D transformed with Ato1 mutants in the residues E144, L219, E144/L219, grown on YNB media containing acetic acid (0.5%, pH 6.0) (AA) or lactic acid (0.5%, pH 5.0) (LA) at 18 °C for 10 days.

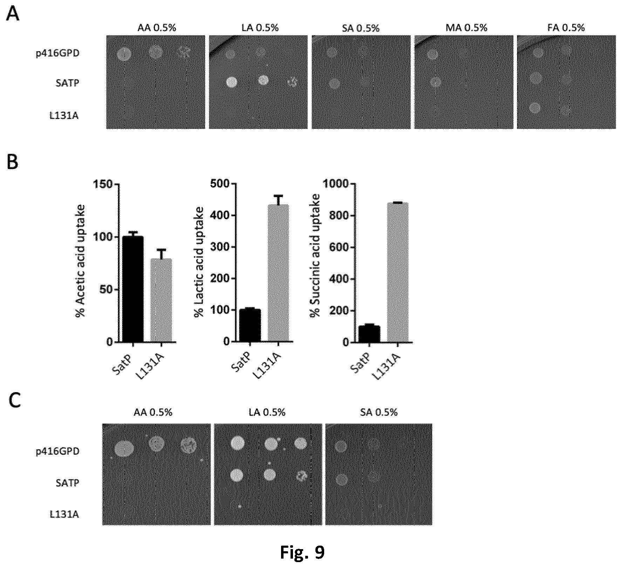

**Figure 9****:** Functional analysis of SatP in S. cerevisiae.
   A - Spot assay of S. cerevisiae IMX1000 transformed with pSatP and pSatP-L131A plasmids. Cells were grown on YNB media containing acetic acid (0.5%, pH 6.0) (AA), lactic acid (0.5%, pH 5.0) (LA), succinic acid (0.5%, pH 5.0) (SA), malic acid (0.5%, pH 5.0) (MA), or fumaric acid (0.5% pH 5.0) (FA), at 18 ºC for 10 days.
   B - Uptake of acetic acid (1.0 mM, pH 6.0), lactic acid (3.0 mM, pH 5.5) and succinic acid (2.0 mM, pH 5.5) in S. cerevisiae IMX1000 cells transformed with the plasmids pSatP and pSatP-L131A. Cells were collected at mid-exponential growth phase from YNB-glucose 2% (w/v), washed and derepressed for 4 h in YNB glycerol (6%).
   C - Spot assay of S. cerevisiae CEN.PK 113-5D strain transformed with p416GPD, pSatP and pSatP-L131A on YNB containing acetic acid (0.5%, pH 6.0) (AA), lactic acid (0.5%, pH 5.0) (LA) or succinic acid (0.5%, pH 5.0) (SA), at 18 °C for 10 days.

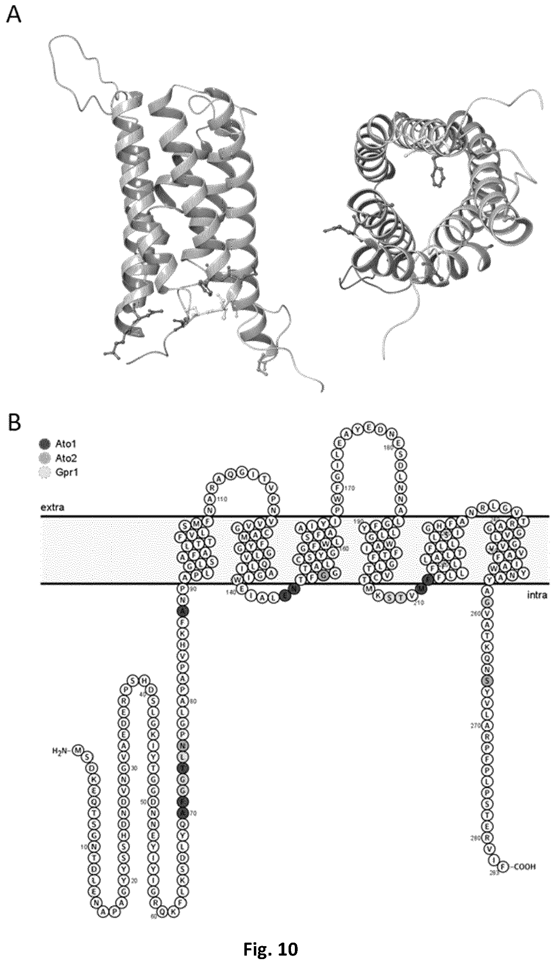

**Figure 10****:** Structural analysis of amino acids from Ato1 (red), Ato2 (orange), and Gpr1 (yellow) whose substitutions led to acetic acid hypersensitivity of yeast.
   A - Location of amino acids whose substitutions led to acetic acid hypersensitivity of yeast within a visualized predicted topology of Ato1.
   B - Location of amino acids whose substitutions led to acetic acid hypersensitivity of yeast within a predicted 3D structure model of Ato1 obtained by homology threading using SatP_Ck crystal structure (PDB Entry: 5YS3) as template.

### DETAILED DESCRIPTION

The present disclosure relates to a yeast cell comprising an expression cassette for expressing a functional SatP transmembrane transporter of organic acids. The present disclosure also relates to a yeast cell comprising an expression cassette for expressing a functional modified SatP and/or Ato1 transmembrane transporter of succinic acid.

The present disclosure also relates to a yeast cell, namely a transformed yeast cell expressing a functional plasma membrane transporter protein for organic acids, in particular succinic acid.

In an embodiment, the *S. cerevisiae* IMX1000 strain was used for characterization of the transporters *ATO1* and *SatP* wild type and mutant alleles, in terms of growth, organic acid uptake, and cellular localization. This strain lacks 25 genes encoding known or putative organic acid transporters and in tested experimental conditions present residual growth on acetic, lactic, pyruvic, succinic, malic and fumaric acid.

In another embodiment, the *S. cerevisiae* CEN.PK 113-5D strain was transformed with the transporters *ATO1* and *SatP* wild type and mutant alleles to assess their dominant negative effect on growth on organic acids. The yeast strains were maintained on solid synthetic medium containing Difco Yeast Nitrogen Base (0.67%, w/v), glucose (2%, w/v) and agar (2%, w/v), supplemented with adequate requirements for prototrophic growth, or on solid YPD medium containing yeast extract (1%, w/v), peptone (2%, w/v), glucose (2%, w/v) and agar (2%, w/v). Plasmids used in this study are listed in Table 1. The p416GPD plasmid with glyceraldehyde-3-phosphate dehydrogenase (GPD) promoter was used as a vector for constitutive expression of *ATO1* and *SatP* wild type and mutant alleles in *S*. *cerevisiae* strains (4,8). The p416GPD contained *URA3* marker gene for auxotrophic complementation (4,8).

**Table 1 List of plasmids used in the different embodiments of the present disclosure.**

| Plasmid | Characteristics | Reference |
|---|---|---|
| p416GPD | p416; Glyceraldehyde-3-phosphate dehydrogenase promoter | 8 |
| pAdy2 | p416GPD derivative; constitutive expression of Ato1 | 4 |
| pAdy2::GFP | p416GPD derivative; constitutive expression of Ato1::GFP | 4 |
| pK86A::GFP | pAdy2::GFP derivative; K86A substitution in Ato1 | |
| pF98A::GFP | pAdy2::GFP derivative; F98A substitution in Ato1 | |
| pQ133A::GFP | pAdy2::GFP derivative; Q133A substitution in Ato1 | |
| pE140A::GFP | pAdy2::GFP derivative; E140A substitution in Ato1 | |
| pE144A::GFP | pAdy2::GFP derivative; E144A substitution in Ato1 | |
| pE144A/F98A::GFP | pAdy2::GFP derivative; E144A and F98A substitutions in Ato1 | |
| pE144A/Y155A::GFP | pAdy2::GFP derivative; E144A and Y155A substitutions in Ato1 | |
| pE144A/L219A::GFP | pAdy2::GFP derivative; E144A and L219A substitutions in Ato1 | |
| pN145A::GFP | pAdy2::GFP derivative; N145A substitution in Ato1 | |
| pT146A::GFP | pAdy2::GFP derivative; T146A substitution in Ato1 | |
| pF147A::GFP | pAdy2::GFP derivative; F147A substitution in Ato1 | |
| pT150A::GFP | pAdy2::GFP derivative; T150A substitution in Ato1 | |
| pL152A::GFP | pAdy2::GFP derivative; L152A substitution in Ato1 | |
| pC153A::GFP | pAdy2::GFP derivative; C153A substitution in Ato1 | |
| pY155A::GFP | pAdy2::GFP derivative; Y155A substitution in Ato1 | |
| pY155S::GFP | pAdy2::GFP derivative; Y155Asubstitution in Ato1 | |
| pY155F::GFP | pAdy2::GFP derivative; Y155F substitution in Ato1 | |
| pF158A::GFP | pAdy2::GFP derivative; F158A substitution in Ato1 | |
| pW159A::GFP | pAdy2::GFP derivative; W159A substitution in Ato1 | |
| pE174A::GFP | pAdy2::GFP derivative; E174A substitution in Ato1 | |
| pE177A::GFP | pAdy2::GFP derivative; E177A substitution in Ato1 | |
| pD178A::GFP | pAdy2::GFP derivative; D178A substitution in Ato1 | |
| pE180A::GFP | pAdy2::GFP derivative; E180A substitution in Ato1 | |
| pD182A::GFP | pAdy2::GFP derivative; D182A substitution in Ato1 | |
| pL191A::GFP | pAdy2::GFP derivative; L191A substitution in Ato1 | |
| pW194A::GFP | pAdy2::GFP derivative; W194A substitution in Ato1 | |
| pF197A::GFP | pAdy2::GFP derivative; F197A substitution in Ato1 | |
| pT198A::GFP | pAdy2::GFP derivative; T198A substitution in Ato1 | |
| pK207A::GFP | pAdy2::GFP derivative; K207A substitution in Ato1 | |
| pF216A::GFP | pAdy2::GFP derivative; F216A substitution in Ato1 | |
| pL219A::GFP | pAdy2::GFP derivative; L219A substitution in Ato1 | |
| pL219V::GFP | pAdy2::GFP derivative; L219V substitution in Ato1 | |
| pL219G::GFP | pAdy2::GFP derivative; L219G substitution in Ato1 | |
| pL219S::GFP | pAdy2::GFP derivative; L219S substitution in Ato1 | |
| pL219P::GFP | pAdy2::GFP derivative; L219P substitution in Ato1 | |
| pL219W::GFP | pAdy2::GFP derivative; L219W substitution in Ato1 | |
| pF223A::GFP | pAdy2::GFP derivative; F223A substitution in Ato1 | |
| pL226A::GFP | pAdy2::GFP derivative; L226A substitution in Ato1 | |
| pH230A::GFP | pAdy2::GFP derivative; H230A substitution in Ato1 | |
| pR239A::GFP | pAdy2::GFP derivative; R239A substitution in Ato1 | |
| pY254A::GFP | pAdy2::GFP derivative; Y254A substitution in Ato1 | |
| pV260A::GFP | pAdy2::GFP derivative; V260A substitution in Ato1 | |
| pT262A::GFP | pAdy2::GFP derivative; T262A substitution in Ato1 | |
| pK263A::GFP | pAdy2::GFP derivative; K263A substitution in Ato1 | |
| pSatP | p416GPD derivative; constitutive expression of SatP | |
| pSatP-L131A | pSatP derivative; L131A substitution in SatP | |

In an embodiment, spot assays were performed by first cultivating the yeast cells in liquid synthetic medium containing Difco Yeast Nitrogen Base (0.67%, w/v) and glucose (2%, w/v), supplemented with adequate requirements for prototrophic growth. Cells were cultivated until mid-exponential phase and diluted to an OD₆₄₀ₙₘ of 0.2. Three 1:10 serial dilutions were made and 3 µl of each suspension were spotted on solid synthetic media containing Difco Yeast Nitrogen Base (0.67%, w/v) and agar (2%, w/v), supplemented with adequate requirements for prototrophic growth. Carbon sources used on solid synthetic media were the following: acetic acid (0.5%, v/v, pH 6.0), lactic acid (0.5%, v/v, pH 5.0, unless specified otherwise), lactic acid (0.5%, v/v, pH 5.0), succinic acid (0.5%, w/v, pH 5.0, unless specified otherwise), malic acid (0.5%, w/v, pH 5.0), fumaric acid (0.5%, w/v, pH 5.0), or glucose (2%, w/v) as control. Yeast cells were cultivated at 18° C for 10 days.

Methods for the alignment of sequences for comparison and protein topology prediction are well known in the art. In an embodiment, to identify conserved amino acid residues within the AceTr family members that are possibly significant for transporter function, protein sequences of AceTr members from *S*. *cerevisiae:* Ato1 (KZV12856.1), Ato2 (KZV08629.1), and Ato3 (KZV12628.1), as well as protein sequences of AceTr members from other organisms that were characterized as acetate transporters: Gpr1 (XP_502188.1), AcpA (Q5B2K4.1), SatP_Ec (AAC73121.1), and SatP_Ck (QQC78103.1) were aligned using the PSI/TM-Coffee server for transmembrane proteins (http://tcoffee.crg.cat/apps/tcoffee/do:tmcoffee). All protein sequences were retrieved from the National Center for Biotechnology Information (NCBI). Topology of Ato1 was predicted and visualised using the Protter program (https://wlab.ethz.ch/protter/star).

In an embodiment, the Ato1 protein sequence was analysed using the HHpred method within the MPI Bioinformatics Toolkit (https://toolkit.tuebingen.mpg.de/) for 3D modelling and HOLE radius prediction (http://www.holeprogram.org) of wild-type Ato1 and its mutants. SatP_Ck [19] was detected as the closest homologue with available crystal structure. By selecting SatP_Ck crystal structure as a template and using the Modeller homology modelling program (https://salilab.org/modeller/), the Ato1 3D structure was obtained. Visual analyses of Ato1 3D structure were performed using the Free Maestro version molecular modelling software as reported by (4).

Using the 3D structures obtained from HHpred, the radius throughout the transporter pore was calculated with the HOLE program (2.2.005 Linux) (http://www.holeprogram.org). The values of the radiuses were displayed in a graph with the X coordinate being the length of the proteins in a determined vector. The Van der Waals radii file used for the predictions was the hard core radii that is supplied by HOLE. The pore predictions were displayed in Visual Molecular Dynamics program (VMD, 1.9.3) along with the 3D structures. The pore predictions were displayed in Visual Molecular Dynamics program (VMD, 1.9.3) along with the 3D structures (http://www.ks.uiuc.edu/Research/vmd/).

In an embodiment, the synthetic *E. coli SatP* wild type gene, with DNA coding sequence optimized for heterologous expression in *S*. *cerevisiae,* was purchased in the form of pUC18-SatP from GenScript (Piscataway, NJ, USA). The codon optimized *SatP* wild type gene was then PCR-amplified from the pUC18-SatP template using the Accuzyme Mix (Bioline, London, UK) and primers SatP_resc_fw and SatP_resc_rew (Table 2). The PCR-amplified *SatP* DNA sequence was cut by *BamH*I and *Xh*oI restriction enzymes (Thermofisher Scientific, Waltham, MA, USA) and cloned into the p416GPD vector, which was digested by the same enzymes under the same conditions, deriving the pSatP.

**Table 2 List of primers used in the different embodiments of the present disclosure.**

| **SEQ ID No** | **Name** | **Sequence** | **Origin** |
|---|---|---|---|
| 1 | K86A fw | CCTGCTCCAGTGCACGCTTTTGCTAATCCTGCGC | Artificial |
| 2 | K86A rev | GCGCAGGATTAGCAAAAGCGTGCACTGGAGCAGG | Artificial |
| 3 | F98A fw | CTTAGGTCTTTCAGCCGCCGCGTTGACGACATTTG | Artificial |
| 4 | F98A rev | CAAATGTCGTCAACGCGGCGGCTGAAAGACCTAAG | Artificial |
| 5 | Q133A fw | GTGGTTTGGTGGCTTTGATTGCTGGTATTTG | Artificial |
| 6 | Q133A rev | CAAATACCAGCAATCAAAGCCACCAAACCAC | Artificial |
| 7 | E140A fw | CTGGTATTTGGGCTATAGCTTTGGAAAATAC | Artificial |
| 8 | E140 rev | GTATTTTCCAAAGCTATAGCCCAAATACCAG | Artificial |
| 9 | E144A fw | GAGATAGCTTTGGCTAATACTTTTGGTGGTAC | Artificial |
| 10 | E144A rev | GTACCACCAAAAGTATTAGCCAAAGCTATCTC | Artificial |
| 11 | N145A fw | GAGATAGCTTTGGAAGCTACTTTTGGTGGTAC | Artificial |
| 12 | N145A rev | GTACCACCAAAAGTAGCTTCCAAAGCTATCTC | Artificial |
| 13 | T146A fw | GATAGCTTTGGAAAATGCTTTTGGTGGTACCG | Artificial |
| 14 | T146A rev | CGGTACCACCAAAAGCATTTTCCAAAGCTATC | Artificial |
| 15 | F147A fw | TTTGGAAAATACTGCTGGTGGTACCGCATTA | Artificial |
| 16 | F147A rev | TAATGCGGTACCACCAGCAGTATTTTCCAAA | Artificial |
| 17 | T150A fw | AATACTTTTGGTGGTGCTGCATTATGTTCTTA | Artificial |
| 18 | T150 rev | TAAGAACATAATGCAGCACCACCAAAAGTATT | Artificial |
| 19 | L152A fw | TTGGTGGTACCGCAGCTTGTTCTTACGGTG | Artificial |
| 20 | L152A rev | CACCGTAAGAACAAGCTGCGGTACCACCAA | Artificial |
| 21 | C153A fw | GGTGGTACCGCATTAGCTTCTTACGGTGGGTTTTG | Artificial |
| 22 | C153A rev | CAAAACCCACCGTAAGAAGCTAATGCGGTACCACC | Artificial |
| 23 | Y155A fw | CATTATGTTCTGCTGGTGGGTTTTGGTTGAG | Artificial |
| 24 | Y155A rev | CTCAACCAAAACCCACCAGCAGAACATAATG | Artificial |
| 25 | Y155S fw | CATTATGTTCTTCTGGTGGGTTTTGGTTGAG | Artificial |
| 26 | Y155S rev | CTCAACCAAAACCCACCAGAAGAACATAATG | Artificial |
| 27 | Y155F fw | CATTATGTTCTTTCGGTGGGTTTTGGTTGAG | Artificial |
| 28 | Y155F rev | CTCAACCAAAACCCACCGAAAGAACATAATG | Artificial |
| 29 | F158A fw | GTTCTTACGGTGGGGCTTGGTTGAGTTTCGCTG | Artificial |
| 30 | F158A rev | CAGCGAAACTCAACCAAGCCCCACCGTAAGAAC | Artificial |
| 31 | W159A fw | CTTACGGTGGGTTTGCTTTGAGTTTCGCTGC | Artificial |
| 32 | W159A rev | GCAGCGAAACTCAAAGCAAACCCACCGTAAG | Artificial |
| 33 | E174A fw | GTTTGGTATCTTGGCTGCTTACGAAGACAATG | Artificial |
| 34 | E174A rev | CATTGTCTTCGTAAGCAGCCAAGATACCAAAC | Artificial |
| 35 | E177A fw | TCTTGGAAGCTTACGCTGACAATGAATCTG | Artificial |
| 36 | E177A rev | CAGATTCATTGTCAGCGTAAGCTTCCAAGA | Artificial |
| 37 | D178A fw | GGAAGCTTACGAAGCTAATGAATCTGATTTG | Artificial |
| 38 | D178A rev | CAAATCAGATTCATTAGCTTCGTAAGCTTCC | Artificial |
| 39 | E180A fw | CTTACGAAGACAATGCTTCTGATTTGAATAATG | Artificial |
| 40 | E180A rev | CATTATTCAAATCAGAAGCATTGTCTTCGTAAG | Artificial |
| 41 | D182A fw | GAAGACAATGAATCTGCTTTGAATAATGCTTTAG | Artificial |
| 42 | D182A rev | CTAAAGCATTATTCAAAGCAGATTCATTGTCTTC | Artificial |
| 43 | L191A fw | GCTTTAGGATTTTATGCTTTGGGGTGGGCCATC | Artificial |
| 44 | L191A rev | GATGGCCCACCCCAAAGCATAAAATCCTAAAGC | Artificial |
| 45 | W194A fw | TTATTTGTTGGGGGCTGCCATCTTTACGTTTG | Artificial |
| 46 | W194A rev | CAAACGTAAAGATGGCAGCCCCCAACAAATAA | Artificial |
| 47 | F197A fw | GTTGGGGTGGGCCATCGCTACGTTTGGTTTAAC | Artificial |
| 48 | F197A rev | GTTAAACCAAACGTAGCGATGGCCCACCCCAAC | Artificial |
| 49 | T198A fw | GGGTGGGCCATCTTTGCTTTTGGTTTAACCG | Artificial |
| 50 | T198A rev | CGGTTAAACCAAAAGCAAAGATGGCCCACCC | Artificial |
| 51 | K207A fw | CCGTTTGTACCATGGCTTCCACTGTTATGTTCTTTTTG | Artificial |
| 52 | K207 rev | CAAAAAGAACATAACAGTGGAAGCCATGGTACAAACGG | Artificial |
| 53 | F216A fw | GTTATGTTCTTTTTGTTGGCTTTCTTACTAGCATTAAC | Artificial |
| 54 | F216A rev | GTTAATGCTAGTAAGAAAGCCAACAAAAAGAACATAAC | Artificial |
| 55 | L219A fw | GTTGTTCTTCTTAGCTGCATTAACTTTCCTACTG | Artificial |
| 56 | L219A rev | CAGTAGGAAAGTTAATGCAGCTAAGAAGAACAAC | Artificial |
| 57 | L219V fw | GTTGTTCTTCTTAGTTGCATTAACTTTCCTACTG | Artificial |
| 58 | L219V rev | CAGTAGGAAAGTTAATGCAACTAAGAAGAACAAC | Artificial |
| 59 | L219G fw | GTTGTTCTTCTTAGGTGCATTAACTTTCCTACTG | Artificial |
| 60 | L219G rev | CAGTAGGAAAGTTAATGCACCTAAGAAGAACAAC | Artificial |
| 61 | L219S fw | GTTGTTCTTCTTATCTGCATTAACTTTCCTACTG | Artificial |
| 62 | L219S rev | CAGTAGGAAAGTTAATGCAGATAAGAAGAACAAC | Artificial |
| 63 | L219P fw | GTTGTTCTTCTTACCAGCATTAACTTTCCTACTG | Artificial |
| 64 | L219P rev | CAGTAGGAAAGTTAATGCTGGTAAGAAGAACAAC | Artificial |
| 65 | L219W fw | GTTGTTCTTCTTATGGGCATTAACTTTCCTACTG | Artificial |
| 66 | L219W rev | CAGTAGGAAAGTTAATGCCCATAAGAAGAACAAC | Artificial |
| 67 | F223A fw | CTTACTAGCATTAACTGCTCTACTGTTGTCTATTGG | Artificial |
| 68 | F223A rev | CCAATAGACAACAGTAGAGCAGTTAATGCTAGTAAG | Artificial |
| 69 | L226A fw | CATTAACTTTCCTACTGGCTTCTATTGGTCACTTTG | Artificial |
| 70 | L226A rev | CAAAGTGACCAATAGAAGCCAGTAGGAAAGTTAATG | Artificial |
| 71 | H230A fw | CTGTTGTCTATTGGTGCTTTTGCTAATAGACTTGG | Artificial |
| 72 | H230 rev | CCAAGTCTATTAGCAAAAGCACCAATAGACAACAG | Artificial |
| 73 | R239A fw | GACTTGGTGTCACAGCTGCTGGTGGTGTCCTGG | Artificial |
| 74 | R239A rev | CCAGGACACCACCAGCAGCTGTGACACCAAGTC | Artificial |
| 75 | Y254A fw | CTTTCATTGCTTGGGCTAACGCATATGCAGG | Artificial |
| 76 | Y254A rev | CCTGCATATGCGTTAGCCCAAGCAATGAAAG | Artificial |
| 77 | V260A fw | CGCATATGCAGGTGTCGCTACAAAGCAGAA | Artificial |
| 78 | V260 rev | TTCTGCTTTGTAGCGACACCTGCATATGCG | Artificial |
| 79 | T262A fw | CGCATATGCAGGTGTTGCTGCTAAGCAGAATTC | Artificial |
| 80 | T262A rev | GAATTCTGCTTAGCAGCAACACCTGCATATGCG | Artificial |
| 81 | K263A fw | GCAGGTGTTGCTACAGCTCAGAATTCATATGTACTG | Artificial |
| 82 | K263A rev | CAGTACATATGAATTCTGAGCTGTAGCAACACCTGC | Artificial |
| 83 | SatP_resc fw | AAAAGGATCCATGGGTAACACCAAATTAGCC | Artificial |
| 84 | SatP_resc rev | TTTTCTCGAGCTAATGACTTTCACCGATAGGCAAG | Artificial |
| 85 | L131A fw | CGTTTTCTTTTCTGCTACAGTTTTGTTCGCTTTG | Artificial |
| 86 | L131A rev | CAAAGCGAACAAAACTGTAGCAGAAAAGAAAACG | Artificial |

In an embodiment, yeast cells were grown overnight in a liquid synthetic medium containing Difco Yeast Nitrogen Base (0.67%, w/v) and glucose (2%, w/v), supplemented with adequate requirements for prototrophic growth. Cells were grown until mid-exponential phase, harvested by centrifugation, washed twice in ice-cold deionized water, and incubated in liquid synthetic media containing lactic acid (0.5%, w/v, pH 5.0) or glycerol (6%, w/v) to alleviate glucose repression. After 4 hours, cells were harvested by centrifugation, washed twice in ice-cold deionized water, and resuspended in ice-cold deionized water to a final concentration of about 15 mg dry wt./mL. Yeast cell suspension (30 µL) was transferred into microtubes containing 0.1 M potassium phosphate buffer (60 µL) with a pH of 6.0, unless specified otherwise. After 2 min of incubation at 26 °C, the transport assay started by adding 10 µL of an aqueous solution of the radiolabelled ¹⁴C -carboxylic acid (pH 6.0, unless specified otherwise), incubated at 26 °C for 30 s (acetic acid) or 60 s (lactic and succinic acids), and stopped by adding cold 100 mM non-labelled acid (100 µL), of equivalent pH. The tubes were centrifuged for 5 min at 13000 rpm, the supernatant was discarded, and the cell pellet was resuspended in ice-cold deionized water (1 mL) and centrifuged again for 5 min at 13000 rpm. The supernatant was discarded, and the cell pellet was resuspended in 1 ml of scintillation liquid (Opti-Phase HiSafe II; LKB FSA Laboratory Supplies, Loughborough, UK). Radioactivity was measured in a PerkinElmer Tri-Carb 4810TR liquid scintillation spectrophotometer with disintegrations per minute correction. The following radioactive labelled substrates were utilized: DL-[U-¹⁴C] lactic acid (specific activity [S.a.] 13000 dpm/nmol), sodium salt (PerkinElmer, Massachusetts, USA), [1-¹⁴C] acetic acid (S.a. 13000 dpm/nmol), sodium salt (GE Healthcare, London, UK) and [1-¹⁴C] succinic acid (Specific activity of 6000 dpm/nmol), sodium salt (Moravek Biochemicals, California, USA). The transport kinetics best fitting the experimental initial uptake rates and the kinetic parameters, were determined by a computer-assisted non-linear regression analysis (GraphPAD Software version 4.00, San Diego, CA, USA).

In an embodiment, site-directed mutagenesis was performed according to (4), using pAdy2, pAdy2::GFP and pSatP as templates (Table 1), and primers that confer respective mutations (Table 2).

In an embodiment, cells were grown overnight in a liquid synthetic medium containing Difco Yeast Nitrogen Base (0.67%, w/v) and glucose (2%, w/v), supplemented with adequate requirements for prototrophic growth. Cells were then washed twice in deionized water and incubated for 4 h in lactic acid. Epifluorescence microscopy analysis was performed by using the Olympus BX63 fluorescence microscope and the cellSens image analysis software.

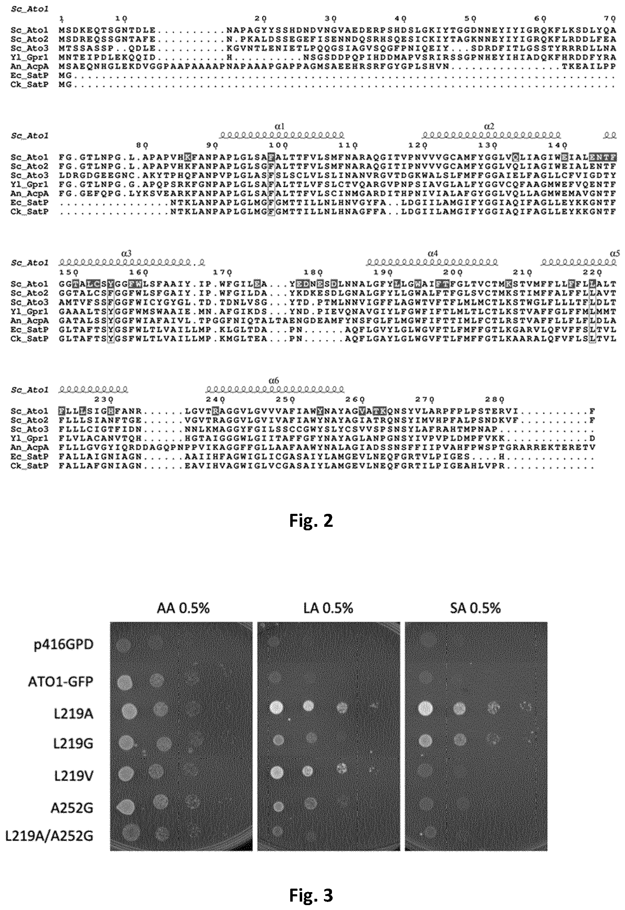

An aspect of the present disclosure relates to the identification of critical residues for the structure and function of *S*. *cerevisiae* Ato1 using a rational site-directed mutagenesis approach. In the first round, a total of 34 mutations (Figure 1A -full spots, Table 1) were generated by substituting selected amino acid residues with an alanine in the ATO1::GFP template cloned in the p416GPD plasmid (4). These amino acid residues were selected as mutagenesis targets by combining the state-of-the-art data with data obtained from primary sequence analysis tools, namely multiple sequence alignment of AceTr homologues functionally characterized as acetate transporters (Figure 2). Furthermore, it was also considered the data obtained from secondary structure-based tools for protein topology prediction allowing the identification of residue location in transmembrane segments, loops or N and C termini (Figure 1A) as well as from computer-assisted three-dimensional modelling tools (Figure 5A).

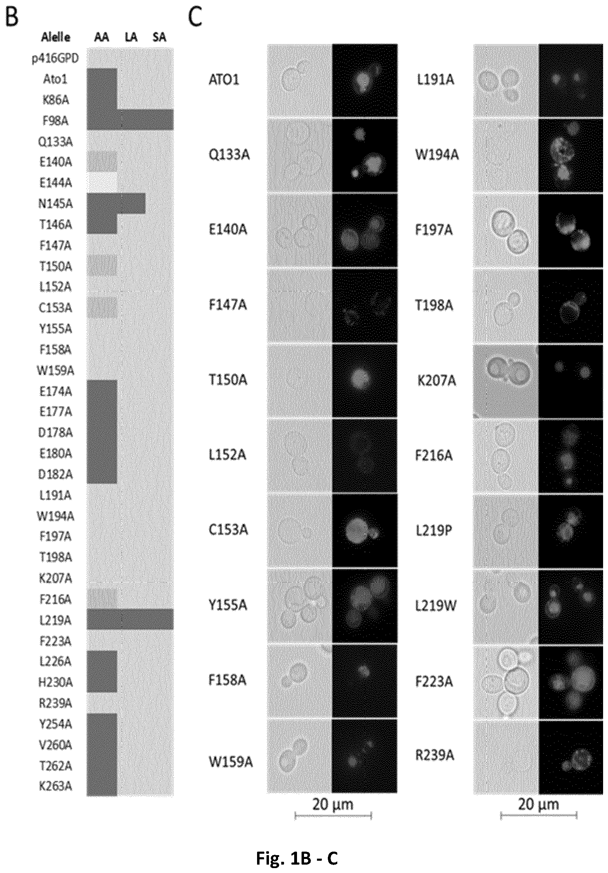

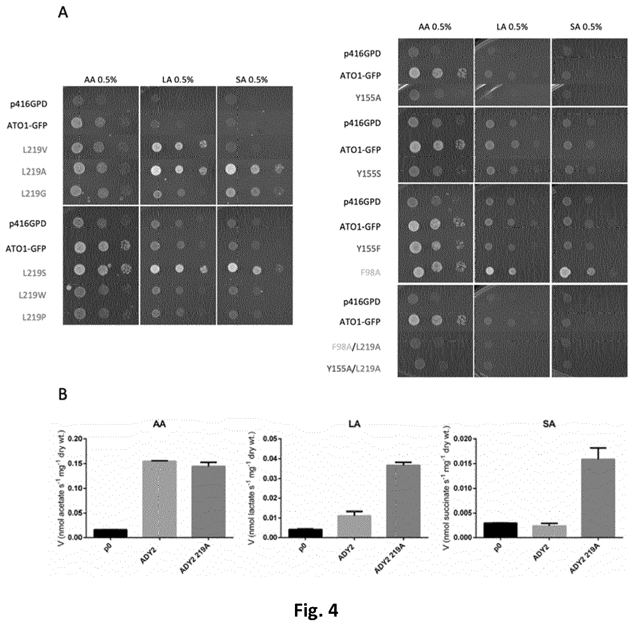

In an embodiment, the phenotype of Ato1 wild type and mutant proteins was evaluated by growth on acetic, lactic or succinic acids as sole carbon and energy sources. According to the phenotypes described in Figure 1B, the mutants could be assigned into distinct categories. The mutants Q133A, E140A, F147A, T150A, L152A, C153A, Y155A, F158A, W159A, L191A, W194A, F197A, T198A, K207A, F216A, F223A, and R239A presented reduced growth on acetic acid. To distinguish whether this phenotype was due to an alteration in the transporter activity or to a deficient protein localization at the plasma membrane, the expression and localization of each of these mutant alleles was analysed by fluorescence microscopy. The mutant transporters E140A and Y155A displayed proper localization at the plasma membrane, whereas the remaining mutants from this group showed absence of GFP labelling at the plasma membrane which is an indication of deficient expression, protein misfolding and/or improper trafficking (Figure 1C), and points towards the involvement of these residues in substrate translocation through Ato1. The N145A mutant, displayed increased ability to grow on lactic acid (Figure 1B). The F98A and L219A mutants presented increased growth both on lactic and on succinic acid, representing a gain of function phenotype by enabling cell growth on succinic acid as a sole carbon source associated with the gain of transport activity for this acid (Figures 3 and 4A). The size of the residue at the position 219 modulates the transporter specificity and activity. By increasing the size of the narrowest constriction in the L219A mutant, the transport of larger molecules, like succinate, becomes possible and the transport of other larger molecules, like lactate, is more efficient (Figure 4B). It was previously shown that the L219V and A252G substitutions improve yeast growth on lactic acid (5), but unlike F98A and L219A, they do not enable growth on succinic acid as a sole carbon source (Figures 3 and 4A). The mutant E144A displayed even lower ability to grow on acetic acid than the strain transformed with the empty vector, which is a sign of hypersensitivity to this acid as reported by (7). Finally, no changes compared to the wild-type were found for K86A, T146A, L226A, H230A, Y254A, V260A, T262A, and K263A.

The Y155A mutant, although properly localized at the plasma membrane, showed only residual growth on acetic acid, similar to cells transformed with p416GPD vector (Figure 4A). The same residual growth was observed in the Y155S mutant. On the other hand, substitution by another aromatic amino acid (Y155F) enabled growth similar to the wild-type Ato1 (Figure 4A). The substitution F98A enabled increased growth on lactic and succinic acids, while keeping growth on acetic acid similar to the wild-type (Figure 4A). The same growth pattern was observed for L219A mutant, a phenotype further confirmed by measuring the uptake for labelled acetic, lactic and succinic acid (Figure 4B), providing evidence for the role of these amino acid residues on transporter specificity and activity. The presence of an aromatic residue at position F98 is not essential for proper function of Atol, whereas an aromatic residue in position Y155 is essential for protein function. Interestingly, excessive modifications of the Ato1 pore, such as the double substitution F98A/L219A (Figure 4A) showed only residual growth on acetic acid and did not allow increased growth on lactic or succinic acids, leading towards the complete abolishment of Ato1 transport activity. The double mutant Y155A/L219A also presented only residual growth on all organic acids tested (Figure 4A).

In another embodiment, Ato1 was mutated by changing the pore size of the narrowest constriction of Ato1 to assess if the activity and specificity of the transporter for lactate or succinate would be altered. The replacements L219A, L219G and L219S allowed growth on lactic and succinic acids, however, the substitution L219V only allowed cells to grow on lactic acid (Figure 4A). These results pointed to the existence of a correlation between the side-chain size of the amino acid at the position 219 and the ability to transport higher molecular weight anions such as: Leucine (acetate) > Valine (acetate and lactate) > Serine > Alanine > Glycine (acetate, lactate, succinate). In yet another embodiment, substitutions L219W and L219P were performed. Due to the absence of growth on carboxylic acids (Figure 4A), these two single mutants were tested for protein localization. The loss of function of the mutant L219P is associated with its improper localization (Figure 1B). However, the L219W was expressed and properly localized at the plasma membrane (Figure 1B), demonstrating that an increased side-chain size at this position decreased the transport capacity, *i.e.* a larger residue results in the blockage of acetate transport through the pore. However, other structural features besides the pore size are also contributing to Ato1 substrate specificity and transport activity, since the substitution Y155A widens the pore but results in the loss of transporter function (Figures 1B and 4A). It was demonstrated that lactate is a substrate of the Ato1 (1), however its transport activity is not sufficient to allow growth on this acid in the conditions used in the present embodiment (Figure 4A). Thus, in Atol, the L129A/G/S/V substitutions increased the capacity of transporting lactate, whereas the L129A/G/S also changed the specificity, enabling the transport of succinate (Figure 4B).

In an embodiment, a 3D model of Ato1 and mutated proteins, using the crystal structure of the bacterial homolog SatP_Ck (PDB 5YS3) as a template, was used to determine the effect of the mutations L219A, L219G, L219S, L219V and L219W on the protein structure. Additionally, the pore size of the predicted structures was analysed using the HOLE software. The pore radius profile varied significantly in the different mutants, in particular at the narrowest constriction formed by the sidechains of the FLY constrictive site, located in the middle of the protein. As observed in Figure 5 B and C, the native Ato1 3D model predicts a radius of 1.11 Å whereas mutations L219A/G/S/V result in an increased pore radius in the narrowest constrictive site from 1.39 to 1.8 Å. On the contrary, the mutation L219W presents a decreased pore radius (0.35 Å) at the constrictive site, that results in the blockage of the pore by the aromatic ring of tryptophan, as observed in the three-dimensional representation of the protein pore (Figures 5B and C). These results confirm the close association between the side-chain size of the amino acid residue at the position 219 and the substrate specificity of Ato1 transporter.

In an embodiment, the E144A substitution in Ato1 causes acetic acid hypersensitivity of *S*. *cerevisiae* (Figure 6), similar to the E144G mutation (7). Surprisingly, when analysing the kinetics of acetic acid transport of cells expressing the E144A mutant, it was observed a much higher activity by the mutant than the wild-type Ato1 (Figure 7) suggesting that hypersensitivity is caused by the overactivity of the transporter. The Vₘₐₓ for acetic acid uptake estimated for the E144A mutant was 0.17 nmol.s⁻¹.mg dry weight⁻¹, which represents a 1.5-fold increase over 0.11 nmol.s⁻¹.mg dry weight⁻¹ estimated for the wild-type strain. The affinity for acetic acid transport increased in the mutant E144A, with a *K*ₘ value of 0.13 mM whereas the value estimated for the cells expressing the wild-type allele is 0.31 mM.

In an embodiment, the kinetic parameters (*K*ₘ and *V*ₘₐₓ) were determined by a computer-assisted non-linear regression analysis (GraphPAD Software version 4.00, San Diego, CA, USA) of the initial uptake rates of the acetic acid as a function of the acid concentration.

In another embodiment, the E144A/Y155A double mutant was generated, because it was previously demonstrated that the single Y155A substitution abolishes acetic acid transport activity of Atol, while still allowing its proper localization. The double mutant E144A/Y155A only presented residual growth on acetic acid comparable to the growth of cells transformed with the empty vector (Figure 6), demonstrating that Ato1 mutants with abolished acetic acid transport activity cannot cause hypersensitivity to this acid. On the other hand, the double mutations E144A/L219A and F98A/E144A could still cause acetic acid hypersensitivity (Figure 6), revealing the cis-dominant effect of the E144A over the other functional alleles.

In another embodiment, the E144A substitution did not cause significant alterations in cell growth on lactic acid 0.5% or succinic acid 0.5% at pH 5.0 when compared to wild-type Ato1 (Figure 1A). The double mutant E144A/L219A displayed hypersensitivity to lactic acid 1.0%, pH 5.0, while the respective single mutants displayed increased growth in these conditions (Figure 8A). The observed loss of growth of the double mutant did not correspond to a loss of function, since the same mutant grew well on lactic acid 0.5%, pH 5.0 (Figure 8A). In fact, at a lower concentration of lactic acid (0.1%), the double mutant exhibited better growth performance than the respective single mutants (Figure 8A). We observed similar concentration-dependent behaviour of the double mutant E144A/L219A on varying concentrations of succinic acid (Figure 8B). These results shows that hyperactive transport can be advantageous for cell growth, as long as the acid metabolism can cope with the increased uptake rates.

In an embodiment, the simultaneous co-expression of the wild-type Ato1 and the hyperactive mutant was tested in *S*. *cerevisiae* CEN.PK 113-5D strain can utilize both acetic and lactic acid as sole carbon and energy sources (Figure 8C). This strain displayed impaired growth on acetic acid when expressing the mutant Ato1 transporter E144A (Figure 8C), even though the wild-type Ato1 coding gene is present in the genome of the cell. When expressing the E144A/L219A mutant, the *S*. *cerevisiae* CEN.PK 113-5D strain displayed impaired growth on acetic and lactic acids (Figure 8C). The observed trans-dominant negative behaviour of *E144A* allele for acetic acid sensitivity is in accordance with a previous report (7) and confirms that the hyperactive organic acid transporter was the cause of hypersensitive phenotypes, since the same phenotypes also occur in strains that co-express wild-type and hyperactive Ato1.

An aspect of the present disclosure relates to the expression of the *E. coli* SatP wild type protein (table 3, sequence ID No 103) in the strain *S*. *cerevisiae* IMX1000, which resulted in the functional expression of the full amino acid sequence of SatP from *E. coli* in *S*. *cerevisiae* without any further modifications. The acetate transporter SatP from *E. coli* shares 36% protein sequence similarity with *S*. *cerevisiae* Ato1 and mediates the uptake of acetate and succinate (5). Surprisingly, when expressed in *S*. *cerevisiae,* the wild type SatP presented a different specificity, mediating the uptake of acetic and lactic acids but not of succinic acid. Indeed, the resultant transformed *S*. *cerevisiae* strain is hypersensitive to acetic acid, similar to the strain expressing Ato1 mutant E144A (Figure 9A). Additionally, SatP expression allowed the *S*. *cerevisiae* IMX1000 strain to grow on medium containing lactic acid, 0.5%, pH 5.0, as a sole carbon source (Figure 9A). Initial uptake rates of acetic acid were increased in yeast cells expressing the wild type SatP transporter, when compared to cells transformed with the corresponding empty vector (Figure 7), further demonstrating that SatP is functional in *S*. *cerevisiae.* The kinetics for acetic acid in *S*. *cerevisiae* cells expressing SatP were a Vₘₐₓ of 0.23 nmol.s⁻¹.mg (dcw)⁻¹ and a *K*ₘ value of 0.09 mM (Figure 7). The expression of the bacterial SatP led to even higher acetic acid uptake rates than the Ato1 E144A mutant (Figure 7).

The kinetic parameters (*K*ₘ and *V*ₘₐₓ) were determined by a computer-assisted non-linear regression analysis (GraphPAD Software version 4.00, San Diego, CA, USA) of the initial uptake rates of the acetic acid as a function of the acid concentration.

In an embodiment, the central constrictive site (FLY) of SatP was also engineered by introducing the L131A substitution (equivalent to L219A in Ato1). This substitution caused hypersensitivity not only to acetic, but also to lactic, succinic, and malic acids, indicating that it generates a hyperactive transporter for these acids (Figure 9A). The same cells did not exhibit any growth differences on fumaric acid compared to the wild-type SatP or the empty vector (Figure 9A). The SatP L131A mutant showed similar uptake of acetic acid and increased uptake of lactic and succinic acids compared to wild type SatP (Figure 9B). Trans-dominant negative hypersensitive phenotypes were present when *S*. *cerevisiae* CEN.PK 113-5D strains expressed SatP and the SatP mutant with L131A substitution (Figure 9C).

In an embodiment, the transport activities of Ato1 E144A mutant and SatP are directly responsible for the observed higher uptake rates of organic acids and causing hypersensitivity of yeast cells to these acids, as observed by the combination of mutations tested.

In another embodiment, the expression of the mutant transporter Ato1 E144A and SatP triggered acetic acid hypersensitivity in a dominant negative manner in *S*. *cerevisiae* (Figures 6 and 9, respectively).

In an embodiment, the hypersensitivity phenotype was extended to other carboxylic acids through the engineering of the central constrictive site (FLY), and consequent increase of substrate specificity and activity of Ato1 E144A/L219A as well as of SatP L131A mutants (Figures 8 and 9, respectively). Cells expressing the Ato1 double E144A/L219A mutant displayed hypersensitivity to lactic and succinic acids in a concentration dependent manner (Figure 8), as the L219A substitution allowed Ato1 E144A/L219A double mutant to import these acids more efficiently. Accordingly, SatP L131A mutant enabled significantly higher uptake rates of lactic and succinic acids compared to SatP and consequently caused the hypersensitivity of yeast cells to these acids (Figure 9B). At lower concentrations of lactic and succinic acids in the culture media, the Ato1 E144A/L219A double mutant expression exhibited a beneficial rather than toxic effect and enabled growth of *S*. *cerevisiae* IMX1000 strain on these acids as sole carbon sources even more than the Ato1 L219A allele (Figure 8). Ato1 becomes hyperactive upon E144A substitution, while the transport level presented by SatP in *S*. *cerevisiae* is already sufficient to promote a toxic effect when cells are grown on acetic acid, 0.5%, pH 6.0. Uptake of organic acids by hyperactive transporter variants from the AceTr family can overwhelm the yeast cells and inhibit their growth, but such uptake can also be advantageous when concentrations of organic acids are low, as it provides the cells with more carbon and energy sources within a given time.

In an embodiment, the locations of the amino acid residues within Ato1 (A70V, F71V, T74I, A88V, E144G, N145D, M211K, and F212S), including the locations of the equivalent amino acids from Ato2 (N75D, G147D, and S265L) and Gpr1, (G62S, G63D, L65Q and G248D) which also led to acetic acid hypersensitivity in yeast, were analysed. According to the predicted 3D model of Ato1 (Figure 10) these amino acids are only found in the cytosolic termini and cytosolic loops. Cytosolic termini and loops of Atol, Ato2 and Gpr1 can play a role in regulating the influx of organic acids from the extracellular medium into the cytosol, possibly by assembling a barrier which slows down or blocks organic acid entry, such as gates. Introduction of mutations which were described as hypersensitive may prevent the assembly of such barrier, and promote the constantly open state of Atol, Ato2 and Gpr1. Alternatively, the cytosolic termini and loops of these transporters may assemble a structure which serves as a binding site for an unknown negative regulator. Being a bacterial transporter, SatP has shorter termini and loops than its eukaryotic homologs. Moreover, SatP contains a glycine residue (G61) instead of glutamate at the position which corresponds to the E144 of Atol, where the E144G substitution caused acetic acid hypersensitivity [23]. These observations explain why the expression of SatP causes acetic acid hypersensitivity in yeast, without the requirement of any further mutation. These data provide strong evidence that the cytosolic termini and loops play an important role in the regulation of transporters from the AceTr family.

In an embodiment, organic acid toxicity occurred due to the hyperactive organic acid uptake via Ato1 E144A mutant and SatP, rather than passive diffusion, since at the tested pH values the diffusion component of the acids is negligible.

Both ATO1 and SatP wild type and mutant proteins were analysed only in terms of organic acid import. As anion channels, these transporters allow the production of high succinic acid titres similarly to SpMae1 and AnDCT-02.

The present disclosure was supported by the strategic programme UID/BIA/04050/2019 funded by Portuguese funds through the FCT-IP, the project TransAcids (PTDC/BIAMIC/5184/2014) funded by FCT-IP and ERDF by COMPETE 2020-POCI and the project EcoAgriFood (NORTE-01-0145-FEDER-000009) supported by NORTE-2020, under the PORTUGAL 2020 Partnership Agreement as well as the European Union's Horizon 2020 research and innovation programme under the Marie Sktodowska-Curie Yeastdoc grant agreement No 764927.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

The following claims further set out particular embodiments of the disclosure.

### REFERENCES

[1] I. Soares-Silva, D. Ribas, M. Sousa-Silva, J. Azevedo-Silva, T. Rendulić, and M. Casal, "Membrane transporters in the bioproduction of organic acids: State of the art and future perspectives for industrial applications," FEMS Microbiol. Lett., vol. 367, no. 15, 2020, doi: 10.1093/femsle/fnaa118.
[2] M. Casal, O. Queirós, G. Talaia, D. Ribas, and S. Paiva, "Carboxylic acids plasma membrane transporters in saccharomyces cerevisiae," Adv. Exp. Med. Biol., vol. 892, pp. 229-251, 2016, doi: 10.1007/978-3-319-25304-6_9.
[3] M. L. A. Jansen, J. J. Heijen, and R. Verwaal, "(12) United States Patent," WO/2013/004670, 2013.
[4] D. Ribas et al., "The acetate uptake transporter family motif 'NPAPLGL(M/S)' is essential for substrate uptake," Fungal Genet. Biol., vol. 122, no. September 2018, pp. 1-10, 2019, doi: 10.1016/j.fgb.2018.10.001.
[5] J. Sá-Pessoa et al., "SATP (YaaH), a succinate-acetate transporter protein in Escherichia coli," Biochem. J., vol. 454, no. 3, 2013, doi: 10.1042/BJ20130412.
[6] B. Qiu et al., "Succinate-acetate permease from Citrobacter koseri is an anion channel that unidirectionally translocates acetate," Cell Res., vol. 28, no. 6, 2018, doi: 10.1038/s41422-018-0032-8.
[7] F. Matthäus and G. Barth, "The Gpr1/Fun34/YaaH Protein Family in the Nonconventional Yeast Yarrowia lipolytica and the Conventional Yeast Saccharomyces cerevisiae," pp. 145-163, 2013, doi: 10.1007/978-3-642-38320-5_7
[8] D. Mumberg, R. Müller, and M. Funk, "Yeast vectors for the controlled expression of heterologous proteins in different genetic backgrounds," Gene, vol. 156, no. 1, 1995, doi: 10.1016/0378-1119(95)00037-7.

**SEQ.LIST**

| **SEQ ID** | **Name** | **Sequence** | **Origin** |
|---|---|---|---|
| 1 | K86A fw | CCTGCTCCAGTGCACGCTTTTGCTAATCCTGCGC | Artificial |
| 2 | K86A rev | GCGCAGGATTAGCAAAAGCGTGCACTGGAGCAGG | Artificial |
| 3 | F98A fw | CTTAGGTCTTTCAGCCGCCGCGTTGACGACATTTG | Artificial |
| 4 | F98A rev | CAAATGTCGTCAACGCGGCGGCTGAAAGACCTAAG | Artificial |
| 5 | Q133A fw | GTGGTTTGGTGGCTTTGATTGCTGGTATTTG | Artificial |
| 6 | Q133A rev | CAAATACCAGCAATCAAAGCCACCAAACCAC | Artificial |
| 7 | E140A fw | CTGGTATTTGGGCTATAGCTTTGGAAAATAC | Artificial |
| 8 | E140 rev | GTATTTTCCAAAGCTATAGCCCAAATACCAG | Artificial |
| 9 | E144A fw | GAGATAGCTTTGGCTAATACTTTTGGTGGTAC | Artificial |
| 10 | E144A rev | GTACCACCAAAAGTATTAGCCAAAGCTATCTC | Artificial |
| 11 | N145A fw | GAGATAGCTTTGGAAGCTACTTTTGGTGGTAC | Artificial |
| 12 | N145A rev | GTACCACCAAAAGTAGCTTCCAAAGCTATCTC | Artificial |
| 13 | T146A fw | GATAGCTTTGGAAAATGCTTTTGGTGGTACCG | Artificial |
| 14 | T146A rev | CGGTACCACCAAAAGCATTTTCCAAAGCTATC | Artificial |
| 15 | F147A fw | TTTGGAAAATACTGCTGGTGGTACCGCATTA | Artificial |
| 16 | F147A rev | TAATGCGGTACCACCAGCAGTATTTTCCAAA | Artificial |
| 17 | T150A fw | AATACTTTTGGTGGTGCTGCATTATGTTCTTA | Artificial |
| 18 | T150 rev | TAAGAACATAATGCAGCACCACCAAAAGTATT | Artificial |
| 19 | L152A fw | TTGGTGGTACCGCAGCTTGTTCTTACGGTG | Artificial |
| 20 | L152A rev | CACCGTAAGAACAAGCTGCGGTACCACCAA | Artificial |
| 21 | C153A fw | GGTGGTACCGCATTAGCTTCTTACGGTGGGTTTTG | Artificial |
| 22 | C153A rev | CAAAACCCACCGTAAGAAGCTAATGCGGTACCACC | Artificial |
| 23 | Y155A fw | CATTATGTTCTGCTGGTGGGTTTTGGTTGAG | Artificial |
| 24 | Y155A rev | CTCAACCAAAACCCACCAGCAGAACATAATG | Artificial |
| 25 | Y155S fw | CATTATGTTCTTCTGGTGGGTTTTGGTTGAG | Artificial |
| 26 | Y155S rev | CTCAACCAAAACCCACCAGAAGAACATAATG | Artificial |
| 27 | Y155F fw | CATTATGTTCTTTCGGTGGGTTTTGGTTGAG | Artificial |
| 28 | Y155F rev | CTCAACCAAAACCCACCGAAAGAACATAATG | Artificial |
| 29 | F158A fw | GTTCTTACGGTGGGGCTTGGTTGAGTTTCGCTG | Artificial |
| 30 | F158A rev | CAGCGAAACTCAACCAAGCCCCACCGTAAGAAC | Artificial |
| 31 | W159A fw | CTTACGGTGGGTTTGCTTTGAGTTTCGCTGC | Artificial |
| 32 | W159A rev | GCAGCGAAACTCAAAGCAAACCCACCGTAAG | Artificial |
| 33 | E174A fw | GTTTGGTATCTTGGCTGCTTACGAAGACAATG | Artificial |
| 34 | E174A rev | CATTGTCTTCGTAAGCAGCCAAGATACCAAAC | Artificial |
| 35 | E177A fw | TCTTGGAAGCTTACGCTGACAATGAATCTG | Artificial |
| 36 | E177A rev | CAGATTCATTGTCAGCGTAAGCTTCCAAGA | Artificial |
| 37 | D178A fw | GGAAGCTTACGAAGCTAATGAATCTGATTTG | Artificial |
| 38 | D178A rev | CAAATCAGATTCATTAGCTTCGTAAGCTTCC | Artificial |
| 39 | E180A fw | CTTACGAAGACAATGCTTCTGATTTGAATAATG | Artificial |
| 40 | E180A rev | CATTATTCAAATCAGAAGCATTGTCTTCGTAAG | Artificial |
| 41 | D182A fw | GAAGACAATGAATCTGCTTTGAATAATGCTTTAG | Artificial |
| 42 | D182A rev | CTAAAGCATTATTCAAAGCAGATTCATTGTCTTC | Artificial |
| 43 | L191A fw | GCTTTAGGATTTTATGCTTTGGGGTGGGCCATC | Artificial |
| 44 | L191A rev | GATGGCCCACCCCAAAGCATAAAATCCTAAAGC | Artificial |
| 45 | W194A fw | TTATTTGTTGGGGGCTGCCATCTTTACGTTTG | Artificial |
| 46 | W194A rev | CAAACGTAAAGATGGCAGCCCCCAACAAATAA | Artificial |
| 47 | F197A fw | GTTGGGGTGGGCCATCGCTACGTTTGGTTTAAC | Artificial |
| 48 | F197A rev | GTTAAACCAAACGTAGCGATGGCCCACCCCAAC | Artificial |
| 49 | T198A fw | GGGTGGGCCATCTTTGCTTTTGGTTTAACCG | Artificial |
| 50 | T198A rev | CGGTTAAACCAAAAGCAAAGATGGCCCACCC | Artificial |
| 51 | K207A fw | CCGTTTGTACCATGGCTTCCACTGTTATGTTCTTTTTG | Artificial |
| 52 | K207 rev | CAAAAAGAACATAACAGTGGAAGCCATGGTACAAACGG | Artificial |
| 53 | F216A fw | GTTATGTTCTTTTTGTTGGCTTTCTTACTAGCATTAAC | Artificial |
| 54 | F216A rev | GTTAATGCTAGTAAGAAAGCCAACAAAAAGAACATAAC | Artificial |
| 55 | L219A fw | GTTGTTCTTCTTAGCTGCATTAACTTTCCTACTG | Artificial |
| 56 | L219A rev | CAGTAGGAAAGTTAATGCAGCTAAGAAGAACAAC | Artificial |
| 57 | L219V fw | GTTGTTCTTCTTAGTTGCATTAACTTTCCTACTG | Artificial |
| 58 | L219V rev | CAGTAGGAAAGTTAATGCAACTAAGAAGAACAAC | Artificial |
| 59 | L219G fw | GTTGTTCTTCTTAGGTGCATTAACTTTCCTACTG | Artificial |
| 60 | L219G rev | CAGTAGGAAAGTTAATGCACCTAAGAAGAACAAC | Artificial |
| 61 | L219S fw | GTTGTTCTTCTTATCTGCATTAACTTTCCTACTG | Artificial |
| 62 | L219S rev | CAGTAGGAAAGTTAATGCAGATAAGAAGAACAAC | Artificial |
| 63 | L219P fw | GTTGTTCTTCTTACCAGCATTAACTTTCCTACTG | Artificial |
| 64 | L219P rev | CAGTAGGAAAGTTAATGCTGGTAAGAAGAACAAC | Artificial |
| 65 | L219W fw | GTTGTTCTTCTTATGGGCATTAACTTTCCTACTG | Artificial |
| 66 | L219W rev | CAGTAGGAAAGTTAATGCCCATAAGAAGAACAAC | Artificial |
| 67 | F223A fw | CTTACTAGCATTAACTGCTCTACTGTTGTCTATTGG | Artificial |
| 68 | F223A rev | CCAATAGACAACAGTAGAGCAGTTAATGCTAGTAAG | Artificial |
| 69 | L226A fw | CATTAACTTTCCTACTGGCTTCTATTGGTCACTTTG | Artificial |
| 70 | L226A rev | CAAAGTGACCAATAGAAGCCAGTAGGAAAGTTAATG | Artificial |
| 71 | H230A fw | CTGTTGTCTATTGGTGCTTTTGCTAATAGACTTGG | Artificial |
| 72 | H230 rev | CCAAGTCTATTAGCAAAAGCACCAATAGACAACAG | Artificial |
| 73 | R239A fw | GACTTGGTGTCACAGCTGCTGGTGGTGTCCTGG | Artificial |
| 74 | R239A rev | CCAGGACACCACCAGCAGCTGTGACACCAAGTC | Artificial |
| 75 | Y254A fw | CTTTCATTGCTTGGGCTAACGCATATGCAGG | Artificial |
| 76 | Y254A rev | CCTGCATATGCGTTAGCCCAAGCAATGAAAG | Artificial |
| 77 | V260A fw | CGCATATGCAGGTGTCGCTACAAAGCAGAA | Artificial |
| 78 | V260 rev | TTCTGCTTTGTAGCGACACCTGCATATGCG | Artificial |
| 79 | T262A fw | CGCATATGCAGGTGTTGCTGCTAAGCAGAATTC | Artificial |
| 80 | T262A rev | GAATTCTGCTTAGCAGCAACACCTGCATATGCG | Artificial |
| 81 | K263A fw | GCAGGTGTTGCTACAGCTCAGAATTCATATGTACTG | Artificial |
| 82 | K263A rev | CAGTACATATGAATTCTGAGCTGTAGCAACACCTGC | Artificial |
| 83 | SatP_resc fw | AAAAGGATCCATGGGTAACACCAAATTAGCC | Artificial |
| 84 | SatP_resc rev | TTTTCTCGAGCTAATGACTTTCACCGATAGGCAAG | Artificial |
| 85 | L131A fw | CGTTTTCTTTTCTG CTACAGTTTTGTTCG CTTTG | Artificial |
| 86 | L131A rev | CAAAGCGAACAAAACTGTAGCAGAAAAGAAAACG | Artificial |
| 87 | Ato1 DNA | ATGTCTGACAAGGAACAAACGAGCGGAAACACAGATTTGGAGAATGCACCAGC AGGATACTATAGTTCCCATGATAACGACGTTAATGGCGTTGCAGAAGATGAAC GTCCATCTCATGATTCGTTGGGCAAGATTTACACTGGAGGTGATAACAATGAAT ATATCTATATTGGGCGTCAAAAGTTTTTGAAGAGCGACTTATACCAAGCCTTTG GTGGTACCTTGAATCCAGGGTTAGCTCCTGCTCCAGTGCACAAATTTGCTAATC CTGCGCCCTTAGGTCTTTCAGCCTTCGCGTTGACGACATTTGTGCTGTCCATGTT CAATGCGAGAGCGCAAGGGATCACTGTTCCTAATGTTGTCGTCGGTTGTGCTAT GTTTTATGGTGGTTTGGTGCAATTGATTGCTGGTATTTGGGAGATAGCTTTGGA AAATACTTTTGGTGGTACCGCATTATGTTCTTACGGTGGGTTTTGGTTGAGTTTC GCTGCAATTTACATTCCTTGGTTTGGTATCTTGGAAGCTTACGAAGACAATGAA TCTGATTTGAATAATGCTTTAGGATTTTATTTGTTGGGGTGGGCCATCTTTACGT TTGGTTTAACCGTTTGTACCATGAAATCCACTGTTATGTTCTTTTTGTTGTTCTTC TTACTAGCATTAACTTTCCTACTGTTGTCTATTGGTCACTTTGCTAATAGACTTG GTGTCACAAGAGCTGGTGGTGTCCTGGGAGTTGTTGTTGCTTTCATTGCTTGGT ACAACGCATATGCAGGTGTTGCTACAAAGCAGAATTCATATGTACTGGCTCGTC CATTCCCATTACCATCTACTGAAAGGGTAATCTTTTAG | Artificial |
| 88 | Ato1 F98A DNA | ATGTCTGACAAGGAACAAACGAGCGGAAACACAGATTTGGAGAATGCACCAGC AGGATACTATAGTTCCCATGATAACGACGTTAATGGCGTTGCAGAAGATGAAC GTCCATCTCATGATTCGTTGGGCAAGATTTACACTGGAGGTGATAACAATGAAT ATATCTATATTGGGCGTCAAAAGTTTTTGAAGAGCGACTTATACCAAGCCTTTG GTGGTACCTTGAATCCAGGGTTAGCTCCTGCTCCAGTGCACAAATTTGCTAATC CTGCGCCCTTAGGTCTTTCAGCCTTCGCGTTGACGACATTTGTGCTGTCCATGTT CAATGCGAGAGCGCAAGGGATCACTGTTCCTAATGTTGTCGTCGGTTGTGCTAT GTTTTATGGTGGTTTGGTGCAATTGATTGCTGGTATTTGGGAGATAGCTTTGGA AAATACTTTTGGTGGTACCGCATTATGTTCTTACGGTGGGTTTTGGTTGAGTTTC GCTGCAATTTACATTCCTTGGTTTGGTATCTTGGAAGCTTACGAAGACAATGAA TCTGATTTGAATAATGCTTTAGGATTTTATTTGTTGGGGTGGGCCATCTTTACGT TTGGTTTAACCGTTTGTACCATGAAATCCACTGTTATGTTCTTTTTGTTGTTCTTC TTACTAGCATTAACTTTCCTACTGTTGTCTATTGGTCACTTTGCTAATAGACTTGG TGTCACAAGAGCTGGTGGTGTCCTGGGAGTTGTTGTTGCTTTCATTGCTTGGTA CAACGCATATGCAGGTGTTGCTACAAAGCAGAATTCATATGTACTGGCTCGTCC ATTCCCATTACCATCTACTGAAAGGGTAATCTTTTAG | Artificial |
| 89 | Ato1 L219A DNA | ATGTCTGACAAGGAACAAACGAGCGGAAACACAGATTTGGAGAATGCACCAGC AGGATACTATAGTTCCCATGATAACGACGTTAATGGCGTTGCAGAAGATGAAC GTCCATCTCATGATTCGTTGGGCAAGATTTACACTGGAGGTGATAACAATGAAT ATATCTATATTGGGCGTCAAAAGTTTTTGAAGAGCGACTTATACCAAGCCTTTG GTGGTACCTTGAATCCAGGGTTAGCTCCTGCTCCAGTGCACAAATTTGCTAATC CTGCGCCCTTAGGTCTTTCAGCCTTCGCGTTGACGACATTTGTGCTGTCCATGTT CAATGCGAGAGCGCAAGGGATCACTGTTCCTAATGTTGTCGTCGGTTGTGCTAT GTTTTATGGTGGTTTGGTGCAATTGATTGCTGGTATTTGGGAGATAGCTTTGGA AAATACTTTTGGTGGTACCGCATTATGTTCTTACGGTGGGTTTTGGTTGAGTTTC GCTGCAATTTACATTCCTTGGTTTGGTATCTTGGAAGCTTACGAAGACAATGAA TCTGATTTGAATAATGCTTTAGGATTTTATTTGTTGGGGTGGGCCATCTTTACGT TTGGTTTAACCGTTTGTACCATGAAATCCACTGTTATGTTCTTTTTGTTGTTCTTC TTAGCTGCATTAACTTTCCTACTGTTGTCTATTGGTCACTTTGCTAATAGACTTGG TGTCACAAGAGCTGGTGGTGTCCTGGGAGTTGTTGTTGCTTTCATTGCTTGGTA CAACGCATATGCAGGTGTTGCTACAAAGCAGAATTCATATGTACTGGCTCGTCC ATTCCCATTACCATCTACTGAAAGGGTAATCTTTTAG | Artificial |
| 90 | Ato1 L219G DNA | ATGTCTGACAAGGAACAAACGAGCGGAAACACAGATTTGGAGAATGCACCAGC AGGATACTATAGTTCCCATGATAACGACGTTAATGGCGTTGCAGAAGATGAAC GTCCATCTCATGATTCGTTGGGCAAGATTTACACTGGAGGTGATAACAATGAAT ATATCTATATTGGGCGTCAAAAGTTTTTGAAGAGCGACTTATACCAAGCCTTTG GTGGTACCTTGAATCCAGGGTTAGCTCCTGCTCCAGTGCACAAATTTGCTAATC CTGCGCCCTTAGGTCTTTCAGCCTTCGCGTTGACGACATTTGTGCTGTCCATGTT CAATGCGAGAGCGCAAGGGATCACTGTTCCTAATGTTGTCGTCGGTTGTGCTAT GTTTTATGGTGGTTTGGTGCAATTGATTGCTGGTATTTGGGAGATAGCTTTGGA AAATACTTTTGGTGGTACCGCATTATGTTCTTACGGTGGGTTTTGGTTGAGTTTC GCTGCAATTTACATTCCTTGGTTTGGTATCTTGGAAGCTTACGAAGACAATGAA TCTGATTTGAATAATGCTTTAGGATTTTATTTGTTGGGGTGGGCCATCTTTACGT TTGGTTTAACCGTTTGTACCATGAAATCCACTGTTATGTTCTTTTTGTTGTTCTTC TTAGGTGCATTAACTTTCCTACTGTTGTCTATTGGTCACTTTGCTAATAGACTTG GTGTCACAAGAGCTGGTGGTGTCCTGGGAGTTGTTGTTGCTTTCATTGCTTGGT ACAACGCATATGCAGGTGTTGCTACAAAGCAGAATTCATATGTACTGGCTCGTC CATTCCCATTACCATCTACTGAAAGGGTAATCTTTTAG | Artificial |
| 91 | Ato1 L219S DNA | ATGTCTGACAAGGAACAAACGAGCGGAAACACAGATTTGGAGAATGCACCAGC AGGATACTATAGTTCCCATGATAACGACGTTAATGGCGTTGCAGAAGATGAAC GTCCATCTCATGATTCGTTGGGCAAGATTTACACTGGAGGTGATAACAATGAAT ATATCTATATTGGGCGTCAAAAGTTTTTGAAGAGCGACTTATACCAAGCCTTTG GTGGTACCTTGAATCCAGGGTTAGCTCCTGCTCCAGTGCACAAATTTGCTAATC CTGCGCCCTTAGGTCTTTCAGCCTTCGCGTTGACGACATTTGTGCTGTCCATGTT CAATGCGAGAGCGCAAGGGATCACTGTTCCTAATGTTGTCGTCGGTTGTGCTAT GTTTTATGGTGGTTTGGTGCAATTGATTGCTGGTATTTGGGAGATAGCTTTGGA AAATACTTTTGGTGGTACCGCATTATGTTCTTACGGTGGGTTTTGGTTGAGTTTC | Artificial |
| | | GCTGCAATTTACATTCCTTGGTTTGGTATCTTGGAAGCTTACGAAGACAATGAA TCTGATTTGAATAATGCTTTAGGATTTTATTTGTTGGGGTGGGCCATCTTTACGT TTGGTTTAACCGTTTGTACCATGAAATCCACTGTTATGTTCTTTTTGTTGTTCTTC TTATCTGCATTAACTTTCCTACTGTTGTCTATTGGTCACTTTGCTAATAGACTTGG TGTCACAAGAGCTGGTGGTGTCCTGGGAGTTGTTGTTGCTTTCATTGCTTGGTA CAACGCATATGCAGGTGTTGCTACAAAGCAGAATTCATATGTACTGGCTCGTCC ATTCCCATTACCATCTACTGAAAGGGTAATCTTTTAG | |
| 92 | Ato1 L219V DNA | ATGTCTGACAAGGAACAAACGAGCGGAAACACAGATTTGGAGAATGCACCAGC AGGATACTATAGTTCCCATGATAACGACGTTAATGGCGTTGCAGAAGATGAAC GTCCATCTCATGATTCGTTGGGCAAGATTTACACTGGAGGTGATAACAATGAAT ATATCTATATTGGGCGTCAAAAGTTTTTGAAGAGCGACTTATACCAAGCCTTTG GTGGTACCTTGAATCCAGGGTTAGCTCCTGCTCCAGTGCACAAATTTGCTAATC CTGCGCCCTTAGGTCTTTCAGCCTTCGCGTTGACGACATTTGTGCTGTCCATGTT CAATGCGAGAGCGCAAGGGATCACTGTTCCTAATGTTGTCGTCGGTTGTGCTAT GTTTTATGGTGGTTTGGTGCAATTGATTGCTGGTATTTGGGAGATAGCTTTGGA AAATACTTTTGGTGGTACCGCATTATGTTCTTACGGTGGGTTTTGGTTGAGTTTC GCTGCAATTTACATTCCTTGGTTTGGTATCTTGGAAGCTTACGAAGACAATGAA TCTGATTTGAATAATGCTTTAGGATTTTATTTGTTGGGGTGGGCCATCTTTACGT TTGGTTTAACCGTTTGTACCATGAAATCCACTGTTATGTTCTTTTTGTTGTTCTTC TTAGTTGCATTAACTTTCCTACTGTTGTCTATTGGTCACTTTGCTAATAGACTTGG TGTCACAAGAGCTGGTGGTGTCCTGGGAGTTGTTGTTGCTTTCATTGCTTGGTA CAACGCATATGCAGGTGTTGCTACAAAGCAGAATTCATATGTACTGGCTCGTCC ATTCCCATTACCATCTACTGAAAGGGTAATCTTTTAG | Artificial |
| 93 | Ato1 E144A DNA | ATGTCTGACAAGGAACAAACGAGCGGAAACACAGATTTGGAGAATGCACCAGC AGGATACTATAGTTCCCATGATAACGACGTTAATGGCGTTGCAGAAGATGAAC GTCCATCTCATGATTCGTTGGGCAAGATTTACACTGGAGGTGATAACAATGAAT ATATCTATATTGGGCGTCAAAAGTTTTTGAAGAGCGACTTATACCAAGCCTTTG GTGGTACCTTGAATCCAGGGTTAGCTCCTGCTCCAGTGCACAAATTTGCTAATC CTGCGCCCTTAGGTCTTTCAGCCTTCGCGTTGACGACATTTGTGCTGTCCATGTT CAATGCGAGAGCGCAAGGGATCACTGTTCCTAATGTTGTCGTCGGTTGTGCTAT GTTTTATGGTGGTTTGGTGCAATTGATTGCTGGTATTTGGGAGATAGCTTTGGC TAATACTTTTGGTGGTACCGCATTATGTTCTTACGGTGGGTTTTGGTTGAGTTTC GCTGCAATTTACATTCCTTGGTTTGGTATCTTGGAAGCTTACGAAGACAATGAA TCTGATTTGAATAATGCTTTAGGATTTTATTTGTTGGGGTGGGCCATCTTTACGT TTGGTTTAACCGTTTGTACCATGAAATCCACTGTTATGTTCTTTTTGTTGTTCTTC TTACTAGCATTAACTTTCCTACTGTTGTCTATTGGTCACTTTGCTAATAGACTTGG TGTCACAAGAGCTGGTGGTGTCCTGGGAGTTGTTGTTGCTTTCATTGCTTGGTA CAACGCATATGCAGGTGTTGCTACAAAGCAGAATTCATATGTACTGGCTCGTCC ATTCCCATTACCATCTACTGAAAGGGTAATCTTTTAG | Artificial |
| 94 | SatP DNA | ATGGGTAACACCAAATTAGCCAATCCAGCCCCTTTAGGTTTGATGGGTTTCGGT ATGACAACTATCTTATTGAACTTGCACAACGTCGGTTACTTCGCTTTGGATGGTA | Artificial |
| | | TCATCTTGGCAATGGGTATTTCTATGGTGGTATCGCTCAAATCTTCGCAGGTTT GTTGGAATACAAGAAAGGCAACACTTTCGGTTTGACTGCTTTTACATCTTATGG TTCATTCTGGTTGACTTTGGTTGCAATCTTGTTGATGCCAAAGTTGGGTTTAACA GATGCTCCAAATGCACAATTTTTGGGTGTTTACTTGGGTTTATGGGGTGTTTTTA CTTTGTTTATGTTTTTCGGTACATTGAAAGGTGCTAGAGTTTTGCAATTCGTTTTC TTTTCTTTGACAGTTTTGTTCGCTTTGTTGGCAATCGGTAACATTGCTGGTAATG CTGCAATTATTCATTTTGCAGGTTGGATTGGTTTGATTTGTGGTGCTTCAGCAAT ATATTTGGCTATGGGTGAAGTCTTGAACGAACAATTTGGTAGAACTGTCTTGCC TATCGGTGAAAGTCATTAG | |
| 95 | SatP L131A DNA | ATGGGTAACACCAAATTAGCCAATCCAGCCCCTTTAGGTTTGATGGGTTTCGGT ATGACAACTATCTTATTGAACTTGCACAACGTCGGTTACTTCGCTTTGGATGGTA TCATCTTGGCAATGGGTATTTTCTATGGTGGTATCGCTCAAATCTTCGCAGGTTT GTTGGAATACAAGAAAGGCAACACTTTCGGTTTGACTGCTTTTACATCTTATGG TTCATTCTGGTTGACTTTGGTTGCAATCTTGTTGATGCCAAAGTTGGGTTTAACA GATGCTCCAAATGCACAATTTTTGGGTGTTTACTTGGGTTTATGGGGTGTTTTTA CTTTGTTTATGTTTTTCGGTACATTGAAAGGTGCTAGAGTTTTGCAATTCGTTTTC TTTTCTGCTACAGTTTTGTTCGCTTTGTTGGCAATCGGTAACATTGCTGGTAATG CTGCAATTATTCATTTTGCAGGTTGGATTGGTTTGATTTGTGGTGCTTCAGCAAT ATATTTGGCTATGGGTGAAGTCTTGAACGAACAATTTGGTAGAACTGTCTTGCC TATCGGTGAAAGTCATTAG | Artificial |
| 96 | Ato1 Protein | MSDKEQTSGNTDLENAPAGYYSSHDNDVNGVAEDERPSHDSLGKIYTGGDNNEYI YIGRQKFLKSDLYQAFGGTLNPGLAPAPVHKFANPAPLGLSAFALTTFVLSMFNARA QGITVPNVVVGCAMFYGGLVQLIAGIWEIALENTFGGTALCSYGGFWLSFAAIYIP WFGILEAYEDNESDLNNALGFYLLGWAIFTFGLTVCTMKSTVMFFLLFFLLALTFLLL SIGHFANRLGVTRAGGVLGVVVAFIAWYNAYAGVATKQNSYVLARPFPLPSTERVI F | Artificial |
| 97 | Ato1 F98A Protein | MSDKEQTSGNTDLENAPAGYYSSHDNDVNGVAEDERPSHDSLGKIYTGGDNNEYI YIGRQKFLKSDLYQAFGGTLNPGLAPAPVHKFANPAPLGLSAAALTTFVLSMFNAR AQGITVPNVVVGCAMFYGGLVQLIAGIWEIALENTFGGTALCSYGGFWLSFAAIYIP WFGILEAYEDNESDLNNALGFYLLGWAIFTFGLTVCTMKSTVMFFLLFFLLALTFLLL SIGHFANRLGVTRAGGVLGVVVAFIAWYNAYAGVATKQNSYVLARPFPLPSTERVI F | Artificial |
| 98 | Ato1 L219A Protein | MSDKEQTSGNTDLENAPAGYYSSHDNDVNGVAEDERPSHDSLGKIYTGGDNNEYI YIGRQKFLKSDLYQAFGGTLNPGLAPAPVHKFANPAPLGLSAFALTTFVLSMFNARA QGITVPNVVVGCAMFYGGLVQLIAGIWEIALENTFGGTALCSYGGFWLSFAAIYIP WFGILEAYEDNESDLNNALGFYLLGWAIFTFGLTVCTMKSTVMFFLLFFLAALTFLLL SIGHFANRLGVTRAGGVLGVVVAFIAWYNAYAGVATKQNSYVLARPFPLPSTERVI F- | Artificial |
| 99 | Ato1 L219G Protein | MSDKEQTSGNTDLENAPAGYYSSHDNDVNGVAEDERPSHDSLGKIYTGGDNNEYI YIGRQKFLKSDLYQAFGGTLNPGLAPAPVHKFANPAPLGLSAFALTTFVLSMFNARA QGITVPNVVVGCAMFYGGLVQLIAGIWEIALENTFGGTALCSYGGFWLSFAAIYIP | Artificial |
| | | WFGILEAYEDNESDLNNALGFYLLGWAIFTFGLTVCTMKSTVMFFLLFFLGALTFLLL SIGHFANRLGVTRAGGVLGVVVAFIAWYNAYAGVATKQNSYVLARPFPLPSTERVI F- | |
| 100 | Ato1 L219S Protein | MSDKEQTSGNTDLENAPAGYYSSHDNDVNGVAEDERPSHDSLGKIYTGGDNNEYI YIGRQKFLKSDLYQAFGGTLNPGLAPAPVHKFANPAPLGLSAFALTTFVLSMFNARA QGITVPNVVVGCAMFYGGLVQLIAGIWEIALENTFGGTALCSYGGFWLSFAAIYIP WFGILEAYEDNESDLNNALGFYLLGWAIFTFGLTVCTMKSTVMFFLLFFLSALTFLLL SIGHFANRLGVTRAGGVLGVVVAFIAWYNAYAGVATKQNSYVLARPFPLPSTERVI F- | Artificial |
| 101 | Ato1 L219V Protein | MSDKEQTSGNTDLENAPAGYYSSHDNDVNGVAEDERPSHDSLGKIYTGGDNNEYI YIGRQKFLKSDLYQAFGGTLNPGLAPAPVHKFANPAPLGLSAFALTTFVLSMFNARA QGITVPNVVVGCAMFYGGLVQLIAGIWEIALENTFGGTALCSYGGFWLSFAAIYIP WFGILEAYEDNESDLNNALGFYLLGWAIFTFGLTVCTMKSTVMFFLLFFLVALTFLLL SIGHFANRLGVTRAGGVLGVVVAFIAWYNAYAGVATKQNSYVLARPFPLPSTERVI F- | Artificial |
| 102 | AT01 E144A Protein | MSDKEQTSGNTDLENAPAGYYSSHDNDVNGVAEDERPSHDSLGKIYTGGDNNEYI YIGRQKFLKSDLYQAFGGTLNPGLAPAPVHKFANPAPLGLSAFALTTFVLSMFNARA QGITVPNVVVGCAMFYGGLVQLIAGIWEIALANTFGGTALCSYGGFWLSFAAIYIP WFGILEAYEDNESDLNNALGFYLLGWAIFTFGLTVCTMKSTVMFFLLFFLLALTFLLL SIGHFANRLGVTRAGGVLGVVVAFIAWYNAYAGVATKQNSYVLARPFPLPSTERVI F | Artificial |
| 103 | SatP Protein | MGNTKLANPAPLGLMGFGMTTILLNLHNVGYFALDGIILAMGIFYGGIAQIFAGLLE YKKGNTFGLTAFTSYGSFWLTLVAILLMPKLGLTDAPNAQFLGVYLGLWGVFTLFM FFGTLKGARVLQFVFFSLTVLFALLAIGNIAGNAAIIHFAGWIGLICGASAIYLAMGEV LNEQFGRTVLPIGESH- | Artificial |
| 104 | SatP L131A Protein | MGNTKLANPAPLGLMGFGMTTILLNLHNVGYFALDGIILAMGIFYGGIAQIFAGLLE YKKGNTFGLTAFTSYGSFWLTLVAILLMPKLGLTDAPNAQFLGVYLGLWGVFTLFM FFGTLKGARVLQFVFFSATVLFALLAIGNIAGNAAIIHFAGWIGLICGASAIYLAMGE VLNEQFGRTVLPIGESH- | Artificial |
| 105 | P416GPD | GACGAAAGGGCCTCGTGATACGCCTATTTTTATAGGTTAATGTCATGATAATAA TGGTTTCTTAGGACGGATCGCTTGCCTGTAACTTACACGCGCCTCGTATCTTTTA ATGATGGAATAATTTGGGAATTTACTCTGTGTTTATTTATTTTTATGTTTTGTATT TGGATTTTAGAAAGTAAATAAAGAAGGTAGAAGAGTTACGGAATGAAGAAAA AAAAATAAACAAAGGTTTAAAAAATTTCAACAAAAAGCGTACTTTACATATATA TTTATTAGACAAGAAAAGCAGATTAAATAGATATACATTCGATTAACGATAAGT AAAATGTAAAATCACAGGATTTTCGTGTGTGGTCTTCTACACAGACAAGATGAA ACAATTCGGCATTAATACCTGAGAGCAGGAAGAGCAAGATAAAAGGTAGTATT TGTTGGCGATCCCCCTAGAGTCTTTTACATCTTCGGAAAACAAAAACTATTTTTT CTTTAATTTCTTTTTTTACTTTCTATTTTTAATTTATATATTTATATTAAAAAATTTA AATTATAATTATTTTTATAGCACGTGATGAAAAGGACCCAGGTGGCACTTTTCG GGGAAATGTGCGCGGAACCCCTATTTGTTTATTTTTCTAAATACATTCAAATATG | Artificial |
| | | TATCCGCTCATGAGACAATAACCCTGATAAATGCTTCAATAATATTGAAAAAGG AAGAGTATGAGTATTCAACATTTCCGTGTCGCCCTTATTCCCTTTTTTGCGGCAT TTTGCCTTCCTGTTTTTGCTCACCCAGAAACGCTGGTGAAAGTAAAAGATGCTG AAGATCAGTTGGGTGCACGAGTGGGTTACATCGAACTGGATCTCAACAGCGGT AAGATCCTTGAGAGTTTTCGCCCCGAAGAACGTTTTCCAATGATGAGCACTTTT AAAGTTCTGCTATGTGGCGCGGTATTATCCCGTATTGACGCCGGGCAAGAGCA ACTCGGTCGCCGCATACACTATTCTCAGAATGACTTGGTTGAGTACTCACCAGT CACAGAAAAGCATCTTACGGATGGCATGACAGTAAGAGAATTATGCAGTGCTG CCATAACCATGAGTGATAACACTGCGGCCAACTTACTTCTGACAACGATCGGAG GACCGAAGGAGCTAACCGCTTTTTTGCACAACATGGGGGATCATGTAACTCGCC TTGATCGTTGGGAACCGGAGCTGAATGAAGCCATACCAAACGACGAGCGTGAC ACCACGATGCCTGTAGCAATGGCAACAACGTTGCGCAAACTATTAACTGGCGA ACTACTTACTCTAGCTTCCCGGCAACAATTAATAGACTGGATGGAGGCGGATAA AGTTGCAGGACCACTTCTGCGCTCGGCCCTTCCGGCTGGCTGGTTTATTGCTGA TAAATCTGGAGCCGGTGAGCGTGGGTCTCGCGGTATCATTGCAGCACTGGGGC CAGATGGTAAGCCCTCCCGTATCGTAGTTATCTACACGACGGGGAGTCAGGCA ACTATGGATGAACGAAATAGACAGATCGCTGAGATAGGTGCCTCACTGATTAA GCATTGGTAACTGTCAGACCAAGTTTACTCATATATACTTTAGATTGATTTAAAA CTTCATTTTTAATTTAAAAGGATCTAGGTGAAGATCCTTTTTGATAATCTCATGA CCAAAATCCCTTAACGTGAGTTTTCGTTCCACTGAGCGTCAGACCCCGTAGAAA AGATCAAAGGATCTTCTTGAGATCCTTTTTTTCTGCGCGTAATCTGCTGCTTGCA AACAAAAAAACCACCGCTACCAGCGGTGGTTTGTTTGCCGGATCAAGAGCTACC AACTCTTTTTCCGAAGGTAACTGGCTTCAGCAGAGCGCAGATACCAAATACTGT CCTTCTAGTGTAGCCGTAGTTAGGCCACCACTTCAAGAACTCTGTAGCACCGCC TACATACCTCGCTCTGCTAATCCTGTTACCAGTGGCTGCTGCCAGTGGCGATAA GTCGTGTCTTACCGGGTTGGACTCAAGACGATAGTTACCGGATAAGGCGCAGC GGTCGGGCTGAACGGGGGGTTCGTGCACACAGCCCAGCTTGGAGCGAACGAC CTACACCGAACTGAGATACCTACAGCGTGAGCTATGAGAAAGCGCCACGCTTCC CGAAGGGAGAAAGGCGGACAGGTATCCGGTAAGCGGCAGGGTCGGAACAGG AGAGCGCACGAGGGAGCTTCCAGGGGGAAACGCCTGGTATCTTTATAGTCCTG TCGGGTTTCGCCACCTCTGACTTGAGCGTCGATTTTTGTGATGCTCGTCAGGGG GGCGGAGCCTATGGAAAAACGCCAGCAACGCGGCCTTTTTACGGTTCCTGGCC TTTTGCTGGCCTTTTGCTCACATGTTCTTTCCTGCGTTATCCCCTGATTCTGTGGA TAACCGTATTACCGCCTTTGAGTGAGCTGATACCGCTCGCCGCAGCCGAACGAC CGAGCGCAGCGAGTCAGTGAGCGAGGAAGCGGAAGAGCGCCCAATACGCAAA CCGCCTCTCCCCGCGCGTTGGCCGATTCATTAATGCAGCTGGCACGACAGGTTT CCCGACTGGAAAGCGGGCAGTGAGCGCAACGCAATTAATGTGAGTTACCTCAC TCATTAGGCACCCCAGGCTTTACACTTTATGCTTCCGGCTCCTATGTTGTGTGGA ATTGTGAGCGGATAACAATTTCACACAGGAAACAGCTATGACCATGATTACGCC AAGCGCGCAATTAACCCTCACTAAAGGGAACAAAAGCTGGAGCTCAGTTTATC ATTATCAATACTCGCCATTTCAAAGAATACGTAAATAATTAATAGTAGTGATTTT | |
| | | CCTAACTTTATTTAGTCAAAAAATTAGCCTTTTAATTCTGCTGTAACCCGTACATG CCCAAAATAGGGGGCGGGTTACACAGAATATATAACATCGTAGGTGTCTGGGT GAACAGTTTATTCCTGGCATCCACTAAATATAATGGAGCCCGCTTTTTAAGCTG GCATCCAGAAAAAAAAAGAATCCCAGCACCAAAATATTGTTTTCTTCACCAACC ATCAGTTCATAGGTCCATTCTCTTAGCGCAACTACAGAGAACAGGGGCACAAAC AGGCAAAAAACGGGCACAACCTCAATGGAGTGATGCAACCTGCCTGGAGTAAA TGATGACACAAGGCAATTGACCCACGCATGTATCTATCTCATTTTCTTACACCTT CTATTACCTTCTGCTCTCTCTGATTTGGAAAAAGCTGAAAAAAAAGGTTGAAAC CAGTTCCCTGAAATTATTCCCCTACTTGACTAATAAGTATATAAAGACGGTAGGT ATTGATTGTAATTCTGTAAATCTATTTCTTAAACTTCTTAAATTCTACTTTTATAGT TAGTCTTTTTTTTAGTTTTAAAACACCAGAACTTAGTTTCGACGGATTCTAGAAC TAGTGGATCCCCCGGGCTGCAGGAATTCGATATCAAGCTTATCGATACCGTCGA CCTCGAGTCATGTAATTAGTTATGTCACGCTTACATTCACGCCCTCCCCCCACAT CCGCTCTAACCGAAAAGGAAGGAGTTAGACAACCTGAAGTCTAGGTCCCTATTT ATTTTTTTATAGTTATGTTAGTATTAAGAACGTTATTTATATTTCAAATTTTTCTTT TTTTTCTGTACAGACGCGTGTACGCATGTAACATTATACTGAAAACCTTGCTTGA GAAGGTTTTGGGACGCTCGAAGGCTTTAATTTGCGGCCGGTACCCAATTCGCCC TATAGTGAGTCGTATTACGCGCGCTCACTGGCCGTCGTTTTACAACGTCGTGAC TGGGAAAACCCTGGCGTTACCCAACTTAATCGCCTTGCAGCACATCCCCCTTTCG CCAGCTGGCGTAATAGCGAAGAGGCCCGCACCGATCGCCCTTCCCAACAGTTG CGCAGCCTGAATGGCGAATGGCGCGACGCGCCCTGTAGCGGCGCATTAAGCGC GGCGGGTGTGGTGGTTACGCGCAGCGTGACCGCTACACTTGCCAGCGCCCTAG CGCCCGCTCCTTTCGCTTTCTTCCCTTCCTTTCTCGCCACGTTCGCCGGCTTTCCC CGTCAAGCTCTAAATCGGGGGCTCCCTTTAGGGTTCCGATTTAGTGCTTTACGG CACCTCGACCCCAAAAAACTTGATTAGGGTGATGGTTCACGTAGTGGGCCATCG CCCTGATAGACGGTTTTTCGCCCTTTGACGTTGGAGTCCACGTTCTTTAATAGTG GACTCTTGTTCCAAACTGGAACAACACTCAACCCTATCTCGGTCTATTCTTTTGA TTTATAAGGGATTTTGCCGATTTCGGCCTATTGGTTAAAAAATGAGCTGATTTAA CAAAAATTTAACGCGAATTTTAACAAAATATTAACGTTTACAATTTCCTGATGCG GTATTTTCTCCTTACGCATCTGTGCGGTATTTCACACCGCATAGGGTAATAACTG ATATAATTAAATTGAAGCTCTAATTTGTGAGTTTAGTATACATGCATTTACTTAT AATACAGTTTTTTAGTTTTGCTGGCCGCATCTTCTCAAATATGCTTCCCAGCCTGC TTTTCTGTAACGTTCACCCTCTACCTTAGCATCCCTTCCCTTTGCAAATAGTCCTC TTCCAACAATAATAATGTCAGATCCTGTAGAGACCACATCATCCACGGTTCTATA CTGTTGACCCAATGCGTCTCCCTTGTCATCTAAACCCACACCGGGTGTCATAATC AACCAATCGTAACCTTCATCTCTTCCACCCATGTCTCTTTGAGCAATAAAGCCGA TAACAAAATCTTTGTCGCTCTTCGCAATGTCAACAGTACCCTTAGTATATTCTCC AGTAGATAGGGAGCCCTTGCATGACAATTCTGCTAACATCAAAAGGCCTCTAGG TTCCTTTGTTACTTCTTCTGCCGCCTGCTTCAAACCGCTAACAATACCTGGGCCCA CCACACCGTGTGCATTCGTAATGTCTGCCCATTCTGCTATTCTGTATACACCCGC AGAGTACTGCAATTTGACTGTATTACCAATGTCAGCAAATTTTCTGTCTTCGAAG | |
| | | AGTAAAAAATTGTACTTGGCGGATAATGCCTTTAGCGGCTTAACTGTGCCCTCC ATGGAAAAATCAGTCAAGATATCCACATGTGTTTTTAGTAAACAAATTTTGGGA CCTAATGCTTCAACTAACTCCAGTAATTCCTTGGTGGTACGAACATCCAATGAAG CACACAAGTTTGTTTGCTTTTCGTGCATGATATTAAATAGCTTGGCAGCAACAG GACTAGGATGAGTAGCAGCACGTTCCTTATATGTAGCTTTCGACATGATTTATCT TCGTTTCCTGCAGGTTTTTGTTCTGTGCAGTTGGGTTAAGAATACTGGGCAATTT CATGTTTCTTCAACACTACATATGCGTATATATACCAATCTAAGTCTGTGCTCCTT CCTTCGTTCTTCCTTCTGTTCGGAGATTACCGAATCAAAAAAATTTCAAAGAAAC CGAAATCAAAAAAAAGAATAAAAAAAAAATGATGAATTGAATTGAAAAGCTGT GGTATGGTGCACTCTCAGTACAATCTGCTCTGATGCCGCATAGTTAAGCCAGCC CCGACACCCGCCAACACCCGCTGACGCGCCCTGACGGGCTTGTCTGCTCCCGGC ATCCGCTTACAGACAAGCTGTGACCGTCTCCGGGAGCTGCATGTGTCAGAGGTT TTCACCGTCATCACCGAAACGCGCGA | |

## Claims

1. A yeast cell comprising an expression cassette for expressing a functional SatP transmembrane transporter of organic acids.

2. The yeast cell according to the previous claim wherein the expression cassette comprises at least a sequence 90% identical to a DNA sequence of the following list: SEQ ID No 94, SEQ ID No 95, or mixtures thereof; preferably 95% identical or identical.

3. The yeast cell according to any of the previous claims comprising a functional transmembrane transporter protein wherein the transmembrane transporter protein comprises at least a sequence 90% identical to a sequence of the following list: SEQ ID No 103, SEQ ID No 104, or mixtures thereof; preferably 95% identical or identical.

4. The yeast cell according to any of the previous claims wherein the transmembrane transporter is arranged to transport organic acids across the yeast cell membrane, wherein the organic acid is selected from a list comprising: acetic acid, lactic acid, pyruvic acid, malic acid, fumaric acid, succinic acid or mixtures thereof; preferably wherein the transmembrane transporter is arranged to transport the organic acids from an exterior of the transformed yeast cell to an interior of said yeast cell or to transport the organic acid from an interior of the transformed yeast cell to an exterior of said yeast cell.

5. A yeast cell comprising an expression cassette for expressing a functional SatP and/or Ato1 transmembrane transporter of succinic acid, preferably SatP as described in any of the previous claims.

6. The yeast cell according to the previous claim wherein the expression cassette comprises at least a DNA sequence 90% identical to a sequence of the following list: SEQ ID No 88, SEQ ID No 89, SEQ ID No 90, SEQ ID No 91, SEQ ID No 92, SEQ ID No 93, SEQ ID No 94, SEQ ID No 95, or mixtures thereof; preferably 95% identical or identical.

7. The yeast cell according to any of the previous claims 5-6 comprising a functional transmembrane transporter protein wherein the transmembrane transporter protein comprises at least a sequence 90% identical to a sequence of the following list: SEQ ID No 97, SEQ ID No 98, SEQ ID No 99, SEQ ID No 100, SEQ ID No 101, SEQ ID No 102, SEQ ID No 103, SEQ ID No 104 or mixtures thereof; preferably 95% identical or identical.

8. The yeast cell according to any of the previous claims wherein the expression cassette is a yeast promotor, an open reading frame and a yeast terminator, preferably wherein the expression cassette is comprised in a plasmid p416GPD.

9. The yeast cell according to any of previous claims wherein the transmembrane transporter is arranged to transport a second organic acid across the yeast cell membrane, wherein the second organic acid is selected from a list comprising: acetic acid, lactic acid, pyruvic acid, malic acid, fumaric acid, succinic acid, or mixtures thereof.

10. The yeast cell according to any of the previous claims wherein the yeast belongs to the genus *Saccharomyces,* preferably *Saccharomyces cerevisiae.*

11. The yeast cell according to any of the previous claims for succinic acid production.

12. Use of a yeast cell comprising a SatP transmembrane transporter as a succinic acid transporter.

13. Use of a yeast cell comprising an Ato1 transmembrane transporter as a succinic acid transporter.

14. Use of a yeast cell comprising a SatP transporter as an improver of succinic acid production.

15. Use of a yeast cell comprising an Ato1 transporter as an improver of succinic acid production.
